(19)

Europäisches Patentamt
European Patent Office
Office européen des brevets

(11) **EP 3 603 506 A1**

(12) **EUROPEAN PATENT APPLICATION**
published in accordance with Art. 153(4) EPC

(43) Date of publication:
**05.02.2020 Bulletin 2020/06**

(51) Int Cl.:
**A61B 5/11** (2006.01)

(21) Application number: **17903590.2**

(22) Date of filing: **30.03.2017**

(86) International application number:
**PCT/JP2017/013485**

(87) International publication number:
**WO 2018/179301 (04.10.2018 Gazette 2018/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Fujitsu Limited**
**Kawasaki-shi, Kanagawa 211-8588 (JP)**

(72) Inventors:
• **HOTTA, Shinji**
**Kawasaki-shi**
**Kanagawa 211-8588 (JP)**

• **MAEDA, Kazuho**
**Kawasaki-shi**
**Kanagawa 211-8588 (JP)**
• **SASAMOTO, Yuki**
**Kawasaki-shi**
**Kanagawa 211-8588 (JP)**
• **INOMATA, Akihiro**
**Kawasaki-shi**
**Kanagawa 211-8588 (JP)**

(74) Representative: **Hoffmann Eitle**
**Patent- und Rechtsanwälte PartmbB**
**Arabellastraße 30**
**81925 München (DE)**

(54) **INFORMATION PROCESSING SYSTEM, INFORMATION PROCESSING DEVICE AND INFORMATION PROCESSING METHOD**

(57) An information processing apparatus (100) acquires measurement information regarding a movement of a patient from a measuring instrument (101). The information processing apparatus (100) detects a walking period (p1-before) and a walking period (p1-after), based on the measurement information. The information processing apparatus (100) specifies a boundary period (p1-center) between the walking period (p1-before) and the walking period (p1-after). The information processing apparatus (100) calculates, based on the measurement information, dissimilarity of a peak amplitude of an vertical acceleration to the walking period (p1-before), and similarity of a peak interval of a vertical acceleration to the walking period (p1-after), in the boundary period (p1-center). The information processing apparatus (100) determines, based on the calculated dissimilarity and similarity, whether or not the boundary period (p1-center) is an abnormal motion period.

FIG. 1

EP 3 603 506 A1

# Description

## TECHNICAL FIELD

The present invention relates to an information processing system, an information processing apparatus, and an information processing method.

## BACKGROUND ART

Conventionally, in a medical field, it is required to grasp a condition of a patient in order to perform an appropriate medical treatment for the patient. However, it is difficult to accurately understand a patient in a limited time such as a medical interview. Therefore, an effective approach is to mount a sensor device to a patient to grasp motion features of the patient in daily life and use the motion features as a clue for diagnosis and treatment. Specifically, a condition of the patient can be grasped by specifying a motion period in which a motion has been performed by the patient based on time-series data obtained from the sensor device, and referring to the motion period and a feature amount in the motion period.

As a prior art, for example, there is a technique in which a degree of dispersion of binarized data obtained by binarizing a feature amount reflecting acceleration components in a longitudinal direction and a vertical direction at each time is calculated, and a moving motion period is decided within a period in which the degree of dispersion exceeds a predetermined threshold. In addition, for example, there is a technique for determining whether a motion is normal walking or abnormal walking in which landing is inappropriate, based on a relationship between a first peak frequency having a maximum power value and a second peak frequency having the second largest power value, which are detected from a frequency spectrum distribution of an acceleration signal. In addition, for example, there is a technique in which a pace of a movement is detected from a peak position appearing in frequency data.

## CITATION LIST

## PATENT DOCUMENT

Patent Document 1: Japanese Laid-open Patent Publication No. 2015-139667
Patent Document 2: Japanese Laid-open Patent Publication No. 2013-059489
Patent Document 3: Japanese Laid-open Patent Publication No. 2011-188901

## SUMMARY OF INVENTION

## TECHNICAL PROBLEM

However, in the prior art, it may be difficult to specify a motion period in which a motion was performed by a patient. For example, in a case where a patient lost his/her balance in a walking motion, an atypical pattern is observed because time-series data obtained from a sensor device mounted to the patient is disturbed. In this case, a period in which the patient was out of balance cannot be specified as a motion period, nor can motion features before and after the patient lost his/her balance be extracted.

In one aspect, it is an object of the present invention to provide an information processing system, an information processing apparatus, and an information processing method capable of improving accuracy of specifying a motion period and a motion feature.

## SOLUTION TO PROBLEM

According to one embodiment, there are proposed an information processing system, an information processing apparatus, and an information processing method, including: acquiring measurement information regarding a movement of a measurement object; detecting, based on the acquired measurement information, a first motion period in which a motion is performed by the measurement object and a second motion period in which a motion is performed by the measurement object; calculating, based on the acquired measurement information, similarity or dissimilarity of each of one or more feature amounts to the detected first motion period and similarity or dissimilarity of each of one or more feature amounts to the detected second motion period, in a boundary period between the first motion period and the second motion period; and determining, based on a calculation result, whether or not the boundary period is a third motion period in which a motion is performed by the measurement object.

## ADVANTAGEOUS EFFECTS OF INVENTION

According to one aspect, it is possible to improve accuracy of specifying a motion period.

## BRIEF DESCRIPTION OF DRAWINGS

FIG. 1 is an explanatory diagram illustrating an example of an information processing method according to an embodiment.
FIG. 2 is an explanatory diagram illustrating an example of an information processing system 200.
FIG. 3 is a block diagram illustrating an exemplary hardware configuration of an information processing apparatus 100.

FIG. 4 is a block diagram illustrating an exemplary hardware configuration of a measuring instrument 101.

FIG. 5 is a block diagram illustrating an exemplary functional configuration of the information processing apparatus 100.

FIG. 6 is an explanatory diagram illustrating an example of time-series data 600 acquired by the information processing apparatus 100.

FIG. 7 is an explanatory diagram illustrating an example in which the information processing apparatus 100 detects a walking period.

FIG. 8 is an explanatory diagram illustrating an example in which the information processing apparatus 100 specifies a boundary period.

FIG. 9 is an explanatory diagram illustrating an example in which the information processing apparatus 100 determines whether or not the boundary period is an abnormal motion period.

FIG. 10 is an explanatory diagram illustrating an example of a boundary period for which likelihood is calculated.

FIG. 11 is an explanatory diagram illustrating a specific example in which the information processing apparatus 100 divides a boundary period 1030.

FIG. 12 is an explanatory diagram (Part 1) illustrating a first example of calculating dissimilarity of first feature amounts.

FIG. 13 is an explanatory diagram (Part 2) illustrating the first example of calculating the dissimilarity of the first feature amounts.

FIG. 14 is an explanatory diagram (Part 3) illustrating the first example of calculating the dissimilarity of the first feature amounts.

FIG. 15 is an explanatory diagram illustrating a second example of calculating dissimilarity of the first feature amounts.

FIG. 16 is an explanatory diagram illustrating an example of selectively using the first example and the second example of calculating the dissimilarity of the first feature amounts.

FIG. 17 is an explanatory diagram illustrating a third example of calculating dissimilarity of the first feature amounts.

FIG. 18 is an explanatory diagram (Part 1) illustrating a first example of calculating similarity of second feature amounts.

FIG. 19 is an explanatory diagram (Part 2) illustrating the first example of calculating the similarity of the second feature amounts.

FIG. 20 is an explanatory diagram (Part 3) illustrating the first example of calculating the similarity of the second feature amounts.

FIG. 21 is an explanatory diagram illustrating a second example of calculating similarity of the second feature amounts.

FIG. 22 is an explanatory diagram (Part 1) illustrating a second example of calculating likelihood.

FIG. 23 is an explanatory diagram (Part 2) illustrating the second example of calculating the likelihood.

FIG. 24 is an explanatory diagram illustrating a third example of calculating likelihood.

FIG. 25 is an explanatory diagram (Part 1) illustrating a fourth example of calculating likelihood.

FIG. 26 is an explanatory diagram (Part 2) illustrating the fourth example of calculating the likelihood.

FIG. 27 is an explanatory diagram (Part 1) illustrating an example of a determination result as to whether or not a boundary period is an abnormal motion period by the information processing apparatus 100.

FIG. 28 is an explanatory diagram (Part 2) illustrating an example of a determination result as to whether or not a boundary period is an abnormal motion period by the information processing apparatus 100.

FIG. 29 is an explanatory diagram (Part 3) illustrating an example of a determination result as to whether or not a boundary period is an abnormal motion period by the information processing apparatus 100.

FIG. 30 is an explanatory diagram (Part 4) illustrating an example of a determination result as to whether or not a boundary period is an abnormal motion period by the information processing apparatus 100.

FIG. 31 is an explanatory diagram (Part 5) illustrating an example of a determination result as to whether or not a boundary period is an abnormal motion period by the information processing apparatus 100.

FIG. 32 is an explanatory diagram (Part 6) illustrating an example of a determination result as to whether or not a boundary period is an abnormal motion period by the information processing apparatus 100.

FIG. 33 is an explanatory diagram (Part 7) illustrating an example of a determination result as to whether or not a boundary period is an abnormal motion period by the information processing apparatus 100.

FIG. 34 is a flowchart illustrating an example of an analysis processing procedure.

DESCRIPTION OF EMBODIMENTS

Hereinafter, embodiments of an information processing system, an information processing apparatus, and an information processing method disclosed herein will be described in detail with reference to the drawings.

(One Example of Information Processing Method According to Embodiment)

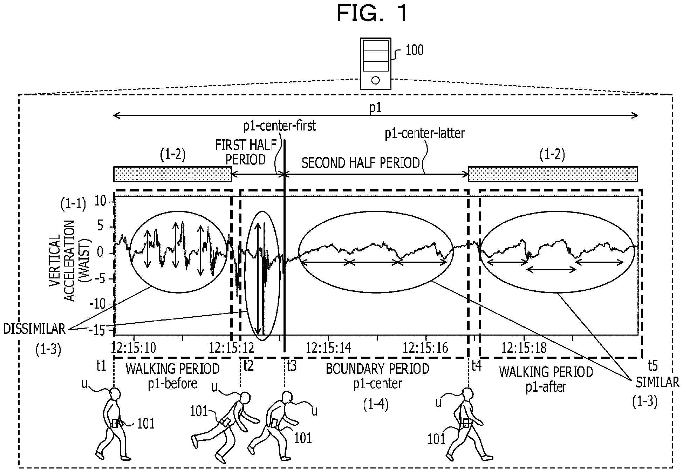

FIG. 1 is an explanatory diagram illustrating an example of an information processing method according to an embodiment. An information processing apparatus 100 is a computer that specifies a motion period in which a motion is performed by a measurement object. The measurement object is a living body. The measurement object may be a machine, for example, an autonomous robot that walks on two legs.

A representative example of the living body is a human. For example, the human is a subject to be examined by a medical institution. Specifically, the human may be a patient who receives a diagnosis, a treatment, rehabilitation instruction, or the like from a medical person such as a doctor. Alternatively, the human may be a trainee who receives motion instruction by a sports instructor. Furthermore, the human may be an individual who performs his/her own health management. Note that the living body is not limited to a human, and may be an animal.

Here, in a motion period in which a predetermined motion is performed by a living body, a characteristic pattern tends to appear in time-series data obtained from a sensor device mounted to the living body. The predetermined motion is, for example, a walking motion. The predetermined motion may be, for example, a standing position maintenance motion, a stair climbing motion, or a cycling motion. The time-series data is, for example, data in which accelerations in a vertical direction of the living body in a certain period are arranged in time series. In the following description, an acceleration in a vertical direction may be referred to as a "vertical acceleration".

The characteristic pattern includes, for example, in a motion period in which a walking motion is performed by a living body, a pattern in which a zero crossing point of a vertical vibration component extracted from an acceleration of the living body appears periodically, or a pattern in which a peak amplitude of a vertical acceleration of the living body appears periodically. The zero crossing point indicates a time point at which a signal value of an object signal becomes zero.

Therefore, there is a case where a characteristic pattern corresponding to a predetermined motion is stored in advance, and a period in which a pattern matching the characteristic pattern appears in time-series data obtained from a sensor device mounted to a living body within a certain period is specified as a motion period of the living body. However, in this case, it may be difficult to specify the motion period of the living body.

For example, when the living body lost its balance in the walking motion, an irregular disturbance may occur in the time-series data obtained from the sensor device mounted to the living body. In addition, in a period in which the living body was out of balance, the pattern matching the characteristic pattern does not appear in the time-series data obtained from the sensor device mounted to the living body, and such a period cannot be specified as a motion period of the living body.

To address this, there is a case where an attempt is made to detect the pattern matching the characteristic pattern even if the irregular disturbance occurs in the time-series data obtained from the sensor device, by extending a range of the pattern determined to match the characteristic pattern before and after a time point at which the characteristic pattern is generated. In this way, there is a case where an attempt is made to accurately specify a motion period of a living body. However, even in this case, it may be difficult to specify the motion period of the living body.

For example, when the living body greatly lost its balance in the walking motion, even if the range of the pattern determined to match the characteristic pattern is extended to the time-series data obtained from the sensor device, a pattern that is difficult to be determined to match the characteristic pattern may appear, for example, in a case where features of a movement suddenly change due to wobble. Therefore, in the period in which the living body was out of balance, the pattern determined to match the characteristic pattern still does not appear in the time-series data obtained from the sensor device mounted to the living body, and such a period cannot be specified as a motion period of the living body.

On the other hand, since the range of the pattern determined to match the characteristic pattern is extended, there is a possibility that a period in which a predetermined motion is not performed by the living body is erroneously specified as a motion period of the living body. In this way, by extending the range of the pattern determined to match the characteristic pattern, there is a possibility that accuracy of specifying a motion period of the living body is lowered.

In addition, since a period in which the living body was out of balance or the like cannot be specified as a motion period of the living body, it is difficult to analyze motion features of the living body in the motion period, and it is difficult for a medical person such as a doctor to grasp the motion features. For example, it is impossible to analyze a time required until a walking way returns after the living body is wobbled, a degree to which a body of the living body is swayed when the living body is wobbled, or the like.

Therefore, in the present embodiment, an information processing method will be described in which, even if no pattern matching a characteristic pattern appears in a certain period, by comparing the period with a period already detected as a motion period, accuracy of specifying a motion period can be improved.

In an example of FIG. 1, a living body is assumed to be a patient. In the example of FIG. 1, the patient performs a walking motion at least in a period p1 from time points t1 to t5. From the time point t1, the patient performs a walking motion in a stable posture. At the time point t2, the patient is out of balance. From the time point t3, the patient is recovering his/her balance and starting to stabilize his/her posture. From the time point t4, the patient performs a walking motion again in a stable posture.

In the example of FIG. 1, the information processing apparatus 100 can communicate with a measuring instrument 101. The measuring instrument 101 measures measurement information regarding a movement of a living body to be a measurement object u. The measuring instrument 101 is mounted to the living body to be the measurement object u. The measuring instrument 101 is mounted to a patient, for example. Specifically, the measuring instrument 101 is a sensor device mounted to the patient at the waist and having an acceleration sensor that measures a vertical acceleration.

(1-1) The information processing apparatus 100 acquires measurement information from the measuring instrument 101. The measurement information is, for example, time-series data in which vertical accelerations measured by the acceleration sensor of the measuring instrument 101 in the period p1 are arranged in time series. The time-series data is, for example, waveform data.

(1-2) The information processing apparatus 100 detects, based on the acquired measurement information, a first motion period in which a predetermined motion is performed by a patient, and a second motion period in which a predetermined motion is performed by the patient. Each of the first motion period and the second motion period is, for example, a walking period in which a walking motion is performed by a patient. The first motion period is before the second motion period, for example.

The information processing apparatus 100 detects, for example, a walking period p1-before from the time point t1 to the time point t2, in which a characteristic pattern corresponding to a walking motion appears in time-series data, in the period p1. In addition, the information processing apparatus 100 detects, for example, a walking period p1-after from the time point t4 to the time point t5, in which a characteristic pattern corresponding to a walking motion appears in time-series data, in the period p1.

Here, the patient is out of balance in a first half period p1-center-first from the time point t2 to the time point t3 of a boundary period p1-center from the time point t2 to the time point t4 between the walking period p1-before and the walking period p1-after. For this reason, it is difficult to detect the boundary period p1-center as a motion period.

Therefore, the information processing apparatus 100 improves accuracy of specifying a motion period by further executing, even in a case where it is difficult to detect the boundary period p1-center as a motion period, processing of determining whether or not the boundary period p1-center is a motion period.

(1-3) The information processing apparatus 100 calculates, based on the acquired measurement information, similarity or dissimilarity of each of one or more feature amounts to the detected first motion period and similarity or dissimilarity of each of one or more feature amounts to the detected second motion period, in the boundary period between the first motion period and the second motion period.

Examples of the one or more feature amounts include at least one of a peak amplitude, a peak interval, a time interval between zero crossing points, an integral value, and a dispersion value for at least one of an acceleration, an angular velocity, a position, and terrestrial magnetism of the measurement object.

In the example of FIG. 1, the information processing apparatus 100 calculates, based on the acquired measurement information, dissimilarity of a first feature amount to the detected first motion period and similarity of a second feature amount to the detected second motion period, in the boundary period between the first motion period and the second motion period.

The first feature amount is a peak amplitude of a vertical acceleration, for example. The peak amplitude is a local maximum value, for example. The peak amplitude may be a local minimum value or a difference between a local maximum value and a local minimum value, for example. The second feature amount is a peak interval of a vertical acceleration, for example. The peak interval is an interval between time points when a local maximum value is reached, for example. The peak interval may be an interval between time points when a local minimum value is reached, for example.

The information processing apparatus 100 calculates, for example, dissimilarity of a peak amplitude of a vertical acceleration to the walking period p1-before, and similarity of a peak interval of a vertical acceleration to the walking period p1-after, in the boundary period p1-center.

Specifically, the information processing apparatus 100 calculates the dissimilarity so that the greater a ratio between a representative value of a peak amplitude of a vertical acceleration in the boundary period p1-center and a representative value of a peak amplitude of a vertical acceleration in the walking period p1-before, the greater the dissimilarity. The representative value is a maximum value, for example. In addition, the representative value may be an average value, a median value, or a minimum value, for example.

Specifically, the information processing apparatus 100 calculates the similarity so that the smaller a ratio between a representative value of a peak interval of a vertical acceleration in the boundary period p1-center and a representative value of a peak interval of a vertical acceleration in the walking period p1-after, the greater the similarity. The representative value is a maximum value, for example. In addition, the representative value may be an average value, a median value, or a minimum value, for example.

Here, there may be a property that, in a case where an abnormal motion related to a walking motion is performed in the boundary period, depending on a type of the abnormal motion, for example, dissimilarity of the first feature amount to the first motion period in the boundary period tends to increase, and similarity of the second feature amount to the second motion period in the boundary period tends to increase. Such an abnormal motion is an exceptional motion in which the patient loses his/her balance by wobble in a walking motion, for example.

The information processing apparatus 100 can use this property to determine whether or not the boundary period is a motion period in which an abnormal motion is performed by the patient, based on the dissimilarity and the similarity. In the following description, a motion period in which an abnormal motion is performed by a patient may be referred to as an "abnormal motion period".

For example, in a case where the patient lost his/her balance, a movement of any part of the patient's body tends to be faster, and then the part tends to receive a strong impact. For example, in a case where the patient lost his/her balance in a walking motion, the patient's foot or waist tends to move faster, and the patient's foot tends to be repelled from a floor the next time the foot lands. In addition, for example, in a case where the patient lost his/her balance in a standing position maintenance motion, the patient's hand or waist may move faster, the patient's hand may strongly press a wall, and the patient's hand may be repelled from the wall.

Therefore, in the boundary period p1-center, when the patient is out of balance, a predetermined feature amount, for example, a peak amplitude of a vertical acceleration tends to include a high-amplitude and high-frequency component, and a representative value of the feature amount tends to become great, as compared with the walking period p1-before. In particular, there is a high possibility that the patient is out of balance in the first half period p1-center-first, and the feature amount in the first half period p1-center-first tends to include a high-amplitude and high-frequency component.

Accordingly, when the patient is out of balance in the boundary period p1-center, dissimilarity between a representative value of a peak amplitude of a vertical acceleration in the boundary period p1-center and a representative value of a peak amplitude of a vertical acceleration in the walking period p1-before tends to be greater. Thus, dissimilarity can be one of criteria for evaluating a degree of possibility that an abnormal motion is performed by the patient, for example, a degree of possibility that the patient lost his/her balance, in the boundary period p1-center.

In addition, the patient tends to recover his/her balance after losing his/her balance. At this time, a movement of any part of the patient's body tends to be slower than before the patient loses his/her balance because the patient is cautious, and the movement tends to gradually return to a movement before the patient loses his/her balance.

For this reason, in the boundary period p1-center, when the patient recovers his/her balance after losing his/her balance, a predetermined feature amount, for example, a representative value of a peak interval of a vertical acceleration tends to be similar to that in the walking period p1-after. In particular, there is a high possibility that the patient recovers his/her balance in a second half period p1-center-latter, and in the second half period p1-center-latter, a predetermined feature amount, for example, a representative value of a peak interval of a vertical acceleration tends to be similar to that in the walking period p1-after.

Accordingly, when the patient recovers his/her balance in the boundary period p1-center, similarity between a representative value of a peak interval of a vertical acceleration in the boundary period p1-center and a representative value of a peak interval of a vertical acceleration in the walking period p1-after tends to be greater. Thus, similarity can be one of criteria for evaluating a degree of possibility that an abnormal motion is performed by the patient, for example, a degree of possibility that the patient lost his/her balance and thereafter recovered his/her balance, in the boundary period p1-center.

(1-4) The information processing apparatus 100 determines whether or not the boundary period is a third motion period in which a motion is performed by the patient, based on the calculated dissimilarity and similarity. The third motion period is an abnormal motion period. The third motion period is, for example, an abnormal motion period in which an abnormal motion related to a walking motion is performed by the patient. Specifically, the third motion period is an abnormal motion period in which an abnormal motion in which the patient lost his/her balance in a walking motion is performed.

For example, the information processing apparatus 100 calculates likelihood of an abnormal motion period based on the dissimilarity and the similarity, and determines whether or not the boundary period p1-center is an abnormal motion period based on the likelihood.

Specifically, the information processing apparatus 100 calculates the likelihood so that the greater the dissimilarity, the greater the likelihood, and the greater the similarity, the greater the likelihood. Then, in a case where the calculated likelihood is equal to or greater than a first threshold, the information processing apparatus 100 determines that the boundary period p1-center is an abnormal motion period. On the other hand, in a case where the calculated likelihood is not equal to or greater than the first threshold, the information processing apparatus 100 determines that the boundary period p1-center is not an abnormal motion period.

In addition, the information processing apparatus 100 may determine whether or not the boundary period p1-center is an abnormal motion period based on, for example, a determination result as to whether or not the dissimilarity is equal to or greater than a second threshold and a determination result as to whether or not the similarity is equal to or greater than a third threshold.

Specifically, in a case where the information processing apparatus 100 determines that the dissimilarity is equal to or greater than the second threshold and determines that the similarity is equal to or greater than the third threshold, the information processing apparatus 100 determines that the boundary period p1-center is an abnormal motion period. On the other hand, in a case where the information processing apparatus 100 determines that the dissimilarity is not equal to or greater than the second threshold, or determines that the similarity is not equal to or greater than the third threshold, the information processing apparatus 100 determines that the boundary period p1-center is not an abnormal motion period.

With this configuration, even if an abnormal motion is performed by the patient and time-series data obtained from the measuring instrument 101 mounted to the patient has a period in which no pattern matching a characteristic pattern appears, the information processing apparatus 100 can determine that the period is an abnormal motion period. In addition, the information processing apparatus 100 does not determine that a period in which an abnormal motion is not performed by the patient is an abnormal motion period, and can suppress a motion period from being erroneously specified. Therefore, the information processing apparatus 100 can improve accuracy of specifying various motion periods including an abnormal motion period.

Thereafter, the information processing apparatus 100 may output a detected motion period, a predetermined feature amount in the motion period, an identified abnormal motion period, a predetermined feature amount in the abnormal motion period, a content of an abnormal motion performed in the abnormal motion period, and the like. The information processing apparatus 100 provides, for example, a detected motion period, a predetermined feature amount in the motion period, an identified abnormal motion period, a predetermined feature amount in the abnormal motion period, and the like to a patient or a medical person such as a doctor. The predetermined feature amount is, for example, any one of a peak amplitude, a peak interval, a time interval between zero crossing points, an integral value, and a dispersion value for any one of an acceleration, an angular velocity, a position, and terrestrial magnetism of a living body.

With this configuration, the information processing apparatus 100 enables a medical person such as a doctor to accurately grasp a condition of the patient based on an abnormal motion period in which an abnormal motion is performed by the patient, a feature amount in the abnormal motion period, a content of the abnormal motion, and the like. In addition, the information processing apparatus 100 can support work of the medical person. Moreover, the information processing apparatus 100 enables the patient or the medical person such as a doctor to accurately grasp features of the abnormal motion of the patient that may lead to a certain disease. As a result, the information processing apparatus 100 can effectively perform health management of the patient, and the like.

For example, the medical person can easily quantify a condition of the patient. In addition, for example, the medical person can easily grasp a condition of the patient more accurately than in a case where an abnormal motion period is not specified. Specifically, in a case where an abnormal motion period is not specified as in the related art, the medical person refers to only a motion period in which the patient does not lose his/her balance, and it is difficult to grasp presence or absence of a condition abnormality in somatic sensation or the like of the patient. On the other hand, the medical person is provided with information regarding an abnormal motion period from the information processing apparatus 100, and can grasp the length and frequency at which the patient lost his/her balance and grasp presence or absence of a condition abnormality in somatic sensation or the like of the patient.

Here, the case where the measuring instrument 101 is mounted to the waist of the patient has been described, but the present invention is not limited to this. For example, the measuring instrument 101 may be mounted to a part other than the waist of the patient. Specifically, the measuring instrument 101 may be mounted to a wrist, ankle, or the like of the patient.

Here, the case where the measuring instrument 101 is mounted to the patient has been described, but the present invention is not limited to this. For example, the measuring instrument 101 may be embedded in a floor on which the patient walks and may measure a pressure applied to the floor from the patient. In addition, for example, the measuring instrument 101 may be installed at a place where an image of the patient can be captured and may measure measurement information regarding a movement of the patient based on the captured image of the patient.

Here, the case where the measuring instrument 101 measures a vertical acceleration has been described, but the present invention is not limited to this. For example, the measuring instrument 101 may measure an acceleration in a horizontal direction. In addition, for example, the measuring instrument 101 may measure an acceleration in a longitudinal direction. In addition, for example, the measuring instrument 101 may measure two or more accelerations among a vertical acceleration, an acceleration in a horizontal direction, and an acceleration in a longitudinal direction. In addition, a type of a measurement signal is not limited to an acceleration, and an angular velocity, terrestrial magnetism, or a position may be measured, for example. In the following description, an acceleration in an horizontal direction may be referred to as a "horizontal acceleration". In the following description, an acceleration in a longitudinal direction may be referred to as a "longitudinal acceleration".

Here, the case has been described where the information processing apparatus 100 calculates the dissimilarity of the first feature amount to the first motion period and the similarity of the second feature amount to the second motion period, in the boundary period, but the present invention is not limited to this. For example, the information processing apparatus 100 may calculate similarity of the first feature amount to the first motion period and dissimilarity of the second feature amount to the second motion period, in the boundary period. In this case, the information processing apparatus 100 calculates likelihood so that the greater the similarity, the smaller the likelihood, and the greater the dissimilarity, the smaller the likelihood.

Here, the case has been described where the information processing apparatus 100 calculates the dissimilarity of the first feature amount to the first motion period in the boundary period, but the present invention is not limited to this. As described above, in the first half period of the boundary period, a high-amplitude and high-frequency component tends to be included. Therefore, for example, the information processing apparatus 100 may calculate dissimilarity of the first feature amount to the first motion period in the first half period of the boundary period.

Here, the case has been described where the information processing apparatus 100 calculates the similarity of the second feature amount to the second motion period in the boundary period, but the present invention is not limited to this. As described above, in the second half period of the boundary period, the second feature amount tends to be similar to that in the second motion period. Therefore, the information processing apparatus 100 may calculate the similarity of the second feature amount to the second motion period in the second half period of the boundary period.

Here, the case has been described where the information processing apparatus 100 determines whether or not the boundary period is an abnormal motion period in which one abnormal motion such as losing balance in a walking motion is performed by the patient, but the present invention is not limited to this. For example, the information processing apparatus 100 may determine whether or not the boundary period is an abnormal motion period in which each abnormal motion of a plurality of abnormal motions is performed. In this case, the information processing apparatus 100 uses a first feature amount and a second feature amount which are different for each abnormal motion.

Examples of the plurality of abnormal motions include, in addition to the above-described abnormal motion such as losing balance in a walking motion, an abnormal motion such as losing balance in a standing position maintenance motion, an abnormal motion such as losing balance in a stair climbing motion, and an abnormal motion such as losing balance in a cycling motion.

Here, the type of the abnormal motion has been described, in which there is a property that the dissimilarity of the first feature amount to the first motion period in the boundary period tends to increase, and the similarity of the second feature amount to the second motion period in the boundary period tends to increase, but the present invention is not limited to this.

For example, there may be a property that, depending on the type of the abnormal motion, the similarity of the first feature amount to the first motion period in the boundary period tends to increase, and the similarity of the second feature amount to the second motion period in the boundary period tends to increase.

In addition, for example, there may be a property that, depending on the type of the abnormal motion, the similarity of the first feature amount to the first motion period in the boundary period tends to increase, and the dissimilarity of the second feature amount to the second motion period in the boundary period tends to increase.

In addition, for example, there may be a property that, depending on the type of the abnormal motion, the dissimilarity of the first feature amount to the first motion period in the boundary period tends to increase, and the dissimilarity of the second feature amount to the second motion period in the boundary period tends to increase.

For example, in a walking motion, there is a case where the patient loses his/her balance because of a temporary headache, and then, after the headache is relieved, returns to the original walk, and there is a case where it is desired to quantify, by using a motion period of such an abnormal motion as a determination object, a feature amount in the motion period. In such a case, for example, a step time per step, that is, a landing interval, is greater in both the first half period and the second half period than in the first motion period and the second motion period, but the difference tends to be relatively small.

Therefore, the information processing apparatus 100 can determine a motion period of such abnormal motion by using a landing interval for both the first feature amount and the second feature amount. For example, in a case where the first feature amount in the first half period is similar to that in the first motion period and the second feature amount in the second half period is similar to that in the second motion period, the information processing apparatus 100 can determine that the boundary period is a motion period of such an abnormal motion.

In addition, for example, in a walking motion, there is a case where, after a foot stumbles over something, the patient may restore a greatly lost balance to restore the original walking, and there is a case where it is desired to quantify, by using a motion period of such an abnormal motion as a determination object, a feature amount in the motion period. In such a case, for example, an amplitude feature of an acceleration of the waist immediately after a stumble, that is, in the first half period, tends to be dissimilar to that in the first motion period, and an amplitude feature of an acceleration of the waist while the patient is restoring a greatly lost balance, that is, in the second half period, also tends to be dissimilar to that in the second motion period.

Therefore, the information processing apparatus 100 can determine a motion period of such an abnormal motion by using an amplitude of an acceleration of the waist for both the first feature amount and the second feature amount. For example, in a case where the first feature amount in the first half period is dissimilar to that in the first motion period and the second feature amount in the second half period is dissimilar to that in the second motion period, the information processing apparatus 100 can determine that the boundary period is a motion period of such an abnormal motion.

In addition, for example, in a stationary motion of supporting a body with one foot, there is a case where, once the patient slightly lost his/her balance, the balance is rapidly lost, and thereafter the patient restores an original stable posture, and there is a case where it is desired to quantify, by using a motion period of such an abnormal motion as a determination object, a feature amount in the motion period. In such a case, for example, amplitude features of an acceleration of the waist tend to be similar between the first motion period and the first half period, that is, a period immediately after a balance is slightly lost, but tend to be dissimilar between the second motion period and the second half period, that is, a period in which a balance is rapidly lost.

Therefore, the information processing apparatus 100 can determine a motion period of such an abnormal motion by using an amplitude of an acceleration of the waist for both the first feature amount and the second feature amount. For example, in a case where the first feature amount in the first half period is similar to that in the first motion period and the second feature amount in the second half period is dissimilar to that in the second motion period, the information processing apparatus 100 can determine that the boundary period is a motion period of such an abnormal motion. In this way, the information processing apparatus 100 can specify which of motion periods of various abnormal motions a boundary period is.

Here, the case has been mainly described where the information processing apparatus 100 uses the first feature amounts in the first motion period and the first half period, but the present invention is not limited to this. For example, as described above, the information processing apparatus 100 may use one or more feature amounts in the first motion period and the first half period.

For example, there is a case where the patient was pushed from a side, slightly wobbled, but kept walking, and there is a case where it is desired to quantify, by using a motion period of such an abnormal motion as a determination object, a feature amount in the motion period. In such a case, in the first half period, an amplitude feature of an acceleration of the waist in a lateral direction tends to be dissimilar to that in the first motion period, but a landing interval tends to be similar to that in the first motion period. Therefore, the information processing apparatus 100 can improve determination accuracy by using dissimilarity of an amplitude feature of an acceleration of the waist in a lateral direction and similarity of a landing interval.

Here, the case has been mainly described where the information processing apparatus 100 uses the second feature amounts in the second motion period and the second half period, but the present invention is not limited to this. For example, as described above, the information processing apparatus 100 may use one or more feature amounts in the second motion period and the second half period.

For example, there is a case where the patient was pushed from behind, wobbled for a few steps, but thereafter held his/her ground and kept walking, and there is a case where it is desired to quantify, by using a motion period of such an abnormal motion as a determination object, a feature amount in the motion period. In such a case, in the second half period, an amplitude feature of an acceleration of the waist in a vertical direction tends to be dissimilar to that in the second motion period, but a landing interval tends to be similar to that in the second motion period. Therefore, the information processing apparatus 100 can improve determination accuracy by using dissimilarity of an amplitude feature of an acceleration of the waist in a vertical direction and a landing interval.

Here, the case has been described where the first motion period and the second motion period are motion periods in which the same motion is performed, but the present invention is not limited to this. For example, the first motion period and the second motion period may be motion periods in which different motions are performed.

(One Example of Information Processing System 200)

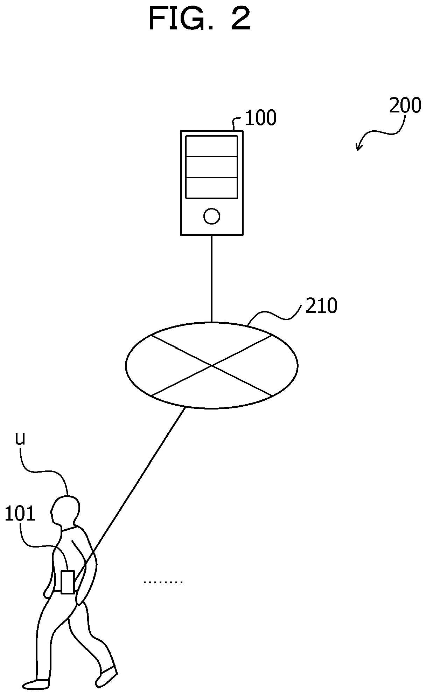

Next, an example of an information processing system 200 to which the information processing apparatus 100 illustrated in FIG. 1 is applied will be described with reference to FIG. 2.

FIG. 2 is an explanatory diagram illustrating an example of the information processing system 200. In FIG. 2, the information processing system 200 includes the information processing apparatus 100 and one or more measuring instruments 101. In the information processing system 200, the information processing apparatus 100 and the measuring instrument 101 are connected via a wired or wireless network 210. Examples of the network 210 include a local area network (LAN), a wide area network (WAN), and the Internet.

The information processing apparatus 100 is a computer that acquires measurement information from the measuring instrument 101, detects a motion period in which a motion is performed by a patient, and specifies an abnormal motion period in which an abnormal motion is performed by the patient. Examples of the information processing apparatus 100 include a server, a personal computer (PC), a notebook PC, a tablet terminal, a smartphone, and a wearable terminal.

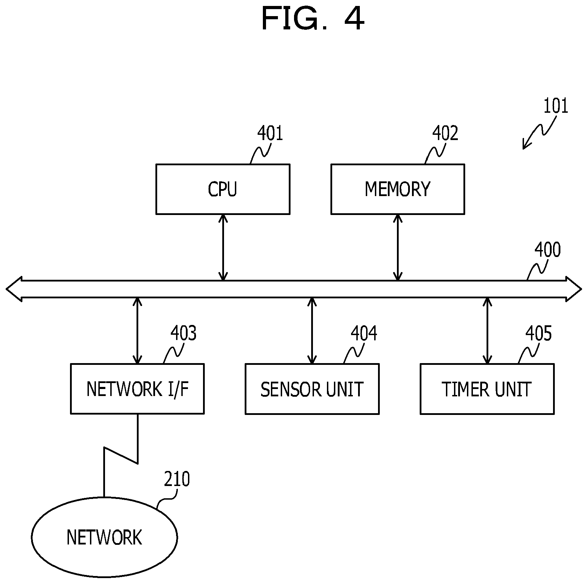

The measuring instrument 101 is a computer mounted to a living body to be a measurement object u. The measuring instrument 101 measures measurement information and transmits the measurement information to the information processing apparatus 100. The measuring instrument 101 includes, for example, a sensor unit illustrated in FIG. 4, creates, as measurement information, time-series data in which a measurement value of the sensor unit and a measurement time at which the measurement value of the sensor unit is obtained are associated with each other, and transmits the time-series data to the information processing apparatus 100. The measuring instrument 101 is a sensor device, for example. The measuring instrument 101 may be a smartphone or a wearable terminal, for example.

Here, the case where the measuring instrument 101 creates time-series data has been described, but the present invention is not limited to this. For example, the information processing apparatus 100 may sequentially receive, from the measuring instrument 101, correspondence information in which a measurement value of the sensor unit of the measuring instrument 101 and a measurement time at which the measurement value of the sensor unit is obtained are associated with each other, and create time-series data in which the correspondence information is compiled.

Here, the case where the information processing apparatus 100 and the measuring instrument 101 are different devices has been described, but the present invention is not limited to this. For example, the information processing apparatus 100 may be integrated with the measuring instrument 101. Here, the case where one measuring instrument 101 is mounted to a living body has been described, but the present invention is not limited to this. For example, a plurality of measuring instruments 101 may be mounted to a living body.

For example, the information processing system 200 is applied to implement a remote watching service or health condition monitoring for grasping a condition of a patient, or is applied to implement a service for a personal health record (PHR).

(Exemplary Hardware Configuration of Information Processing Apparatus 100)

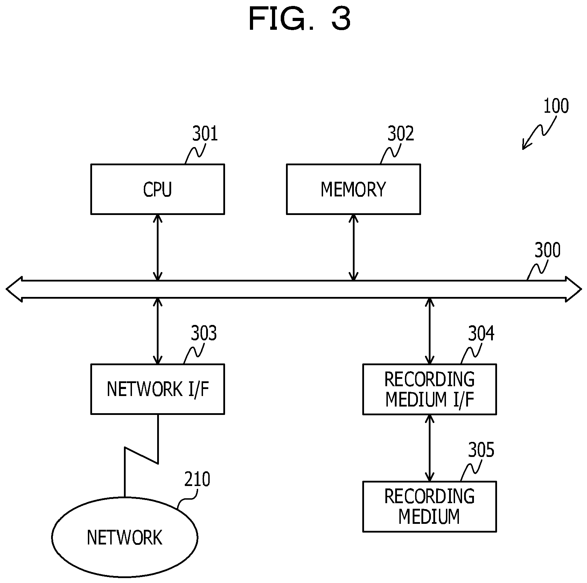

Next, an exemplary hardware configuration of the information processing apparatus 100 included in the information processing system 200 illustrated in FIG. 2 will be described with reference to FIG. 3.

FIG. 3 is a block diagram illustrating an exemplary hardware configuration of the information processing apparatus 100. In FIG. 3, the information processing apparatus 100 includes a central processing unit (CPU) 301, a memory 302, a network interface (I/F) 303, a recording medium I/F 304, and a recording medium 305. In addition, each of components is interconnected by a bus 300.

Here, the CPU 301 performs overall control of the information processing apparatus 100. The memory 302 includes a read only memory (ROM), a random access memory (RAM), and a flash ROM, for example. Specifically, for example, the flash ROM or the ROM stores various programs, while the RAM is used as a work area for the CPU 301. A program stored in the memory 302 is loaded into the CPU 301 to cause the CPU 301 to execute coded processing.

The network I/F 303 is connected to the network 210 through a communication line, and is connected to another computer through the network 210. In addition, the network I/F 303 manages an interface between the network 210 and an inside, and controls input-output of data from another computer. Examples of the network I/F 303 include a modem or a LAN adapter.

The recording medium I/F 304 controls read and write of data toward the recording medium 305 under the control of the CPU 301. Examples of the recording medium I/F 304 include a disk drive, a solid state drive (SSD), or a universal serial bus (USB) port. The recording medium 305 is a nonvolatile memory that stores data written under the control of the recording medium I/F 304. Examples of the recording medium 305 include a disk, a semiconductor memory, or a USB memory. The recording medium 305 may be removably installed on the information processing apparatus 100.

The information processing apparatus 100 may further include a keyboard, a mouse, a display, a printer, a speaker, or a touch panel, for example, in addition to the components described above. In addition, the information processing apparatus 100 may omit the recording medium I/F 304 or the recording medium 305.

(Exemplary Hardware Configuration of Measuring Instrument 101)

Next, an exemplary hardware configuration of the measuring instrument 101 included in the information processing system 200 illustrated in FIG. 2 will be described with reference to FIG. 4.

FIG. 4 is a block diagram illustrating an exemplary hardware configuration of the measuring instrument 101. In FIG. 4, the measuring instrument 101 includes a CPU 401, a memory 402, a network I/F 403, a sensor unit 404, and a timer unit 405. In addition, each of components is interconnected by a bus 400.

Here, the CPU 401 performs overall control of the measuring instrument 101. The memory 402 includes, for example, a ROM, a RAM, and a flash ROM. Specifically, for example, the flash ROM or the ROM stores various programs, while the RAM is used as a work area for the CPU 401. A program stored in the memory 402 is loaded into the CPU 401 to cause the CPU 401 to execute coded processing.

The network I/F 403 is connected to the network 210 through a communication line, and is connected to another computer through the network 210. In addition, the network I/F 403 manages an interface between the network 210 and an inside, and controls input-output of data from another computer. Examples of the network I/F 403 include a communication circuit corresponding to Wi-Fi (registered trademark), or a communication circuit corresponding to Bluetooth (registered trademark). The network I/F 403 may be, for example, a communication circuit corresponding to the 3rd Generation (3G).

The sensor unit 404 measures a condition of the measuring instrument 101. The sensor unit 404 measures, for example, at least one of a position, a movement, and an orientation of the measuring instrument 101. Specifically, the sensor unit 404 includes at least one of an acceleration sensor, an angular velocity sensor, a terrestrial magnetism sensor, an optical sensor, a vibration sensor, and the like. In addition, the sensor unit 404 may include a global positioning systems (GPS) receiver, and may detect GPS coordinates of the measuring instrument 101. The timer unit 405 measures a current time.

The measuring instrument 101 may further include a keyboard, a mouse, a display, a printer, a speaker, or a touch panel, for example, in addition to the components described above. In addition, the measuring instrument 101 may further include a recording medium I/F or a recording medium, in addition to the components described above. The recording medium may be removably installed on the measuring instrument 101.

(Exemplary Functional Configuration of Information Processing Apparatus 100)

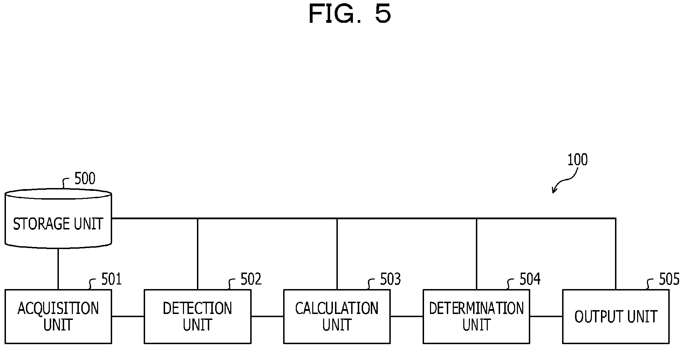

Next, an exemplary functional configuration of the information processing apparatus 100 will be described with reference to FIG. 5.

FIG. 5 is a block diagram illustrating an exemplary functional configuration of the information processing apparatus 100. The information processing apparatus 100 includes a storage unit 500, an acquisition unit 501, a detection unit 502, a calculation unit 503, a determination unit 504, and an output unit 505.

For example, the storage unit 500 is implemented by a storage area such as the memory 302 or the recording medium 305 illustrated in FIG. 3. The acquisition unit 501 to the output unit 505 have functions as a control unit. Specifically, for example, the acquisition unit 501 to the output unit 505 implement functions thereof by causing the CPU 301 to execute a program stored in a storage area such as the memory 302 or the recording medium 305, or by the network I/F 303, illustrated in FIG. 3. A processing result of individual functional units is stored in a storage area such as the memory 302 or the recording medium 305, illustrated in FIG. 3, for example.

The storage unit 500 stores a detection pattern of each motion of a plurality of motions. The motion is, for example, a walking motion, a standing position maintenance motion, a stair climbing motion, or a cycling motion. The detection pattern of a motion defines, for example, a detection condition for detecting a motion period in which the motion is performed. The detection condition indicates, for example, that a characteristic pattern appears for a predetermined feature amount. Specifically, the detection condition indicates that a characteristic pattern in which a zero crossing point periodically appears for a vertical vibration component extracted from an acceleration in three axes appears. With this configuration, the storage unit 500 can enable the detection unit 502 to refer to the detection pattern corresponding to each motion.

The storage unit 500 stores a determination pattern of each abnormal motion of a plurality of abnormal motions. Examples of the plurality of abnormal motions include an abnormal motion in which a living body loses its balance in a walking motion, an abnormal motion in which a living body loses its balance in a standing position maintenance motion, an abnormal motion in which a living body loses its balance in a stair climbing motion, and an abnormal motion in which a living body loses its balance in a cycling motion. The determination pattern of an abnormal motion defines, for example, a determination condition for determining whether or not a boundary period is an abnormal motion period in which the abnormal motion is performed. In addition, the determination pattern of an abnormal motion defines, for example, a first feature amount and a second feature amount used for the determination condition. The determination condition indicates, for example, that likelihood of an abnormal motion period calculated based on the first feature amount and the second feature amount is equal to or greater than a threshold.

The first feature amount is a dispersion value of a synthetic acceleration of a living body, for example. The first feature amount is a peak amplitude of a vertical acceleration of a living body, for example. The first feature amount is a peak amplitude of a longitudinal acceleration of a living body, for example. The first feature amount is a peak amplitude of a horizontal acceleration of a living body. The second feature amount is a dispersion value of a synthetic acceleration of a living body, for example. The second feature amount is a peak interval of a vertical acceleration of a living body, for example. The second feature amount is a peak interval of a longitudinal acceleration of a living body, for example. The second feature amount is a dispersion value of a horizontal acceleration of a living body. With this configuration, the storage unit 500 can enable the determination unit 504 to refer to the determination pattern corresponding to each abnormal motion.

The storage unit 500 stores measurement information regarding a movement of a living body. The storage unit 500 stores, for example, time-series data in which a measurement value of the sensor unit of the measuring instrument 101 and a measurement time at which the measurement value is obtained are associated with each other and arranged in time series. The measurement value is at least one of a vertical acceleration, a horizontal acceleration, or a longitudinal acceleration, for example. The measurement value may be an angular velocity, magnitude of vibration, or a position, for example. With this configuration, the storage unit 500 can enable the detection unit 502 to refer to time-series data for a living body in order to detect a motion period in which a motion is performed by the living body. In addition, the storage unit 500 can enable the calculation unit 503 to refer to the time-series data for the living body.

The acquisition unit 501 acquires measurement information. For example, as measurement information created by the measuring instrument 101, the acquisition unit 501 receives, from the measuring instrument 101, time-series data in which a measurement value of the sensor unit of the measuring instrument 101 and a measurement time at which the measurement value is obtained are associated with each other, and stores the time-series data in the storage unit 500. For example, the acquisition unit 501 may sequentially receive, from the measuring instrument 101, correspondence information in which a measurement value of the sensor unit of the measuring instrument 101 and a measurement time at which the measurement value is obtained are associated with each other, create time-series data in which the correspondence information is compiled, and store the time-series data in the storage unit 500. With this configuration, the acquisition unit 501 can store time-series data for a living body in the storage unit 500 so that the detection unit 502 or the calculation unit 503 can refer to the time-series data.

The detection unit 502 detects a motion period in which a motion is performed by a living body based on the measurement information acquired by the acquisition unit 501. For example, the detection unit 502 refers to the detection pattern stored in the storage unit 500, and detects, as a motion period, each period of a plurality of periods satisfying a detection condition. Specifically, the detection unit 502 detects, as a motion period, each period of a plurality of periods in which a characteristic pattern in which a zero crossing point periodically appears for a vertical vibration component extracted from an acceleration in three axes appears. With this configuration, the detection unit 502 can notify the calculation unit 503 of a motion period used when a boundary period is specified.

The calculation unit 503 selects a first motion period and a second motion period from a plurality of motion periods detected by the detection unit 502, and specifies a boundary period between the first motion period and the second motion period. The first motion period is before the second motion period, for example. Preferably, the first motion period and the second motion period do not include any other motion period between the first motion period and the second motion period. With this configuration, the calculation unit 503 can specify the boundary period that may be an abnormal motion period.

Next, the calculation unit 503 calculates, based on the measurement information acquired by the acquisition unit 501, similarity or dissimilarity of each of one or more feature amounts to the first motion period and similarity or dissimilarity of each of one or more feature amounts to the second motion period, in the specified boundary period.

Examples of the one or more feature amounts include at least one of a peak amplitude, a peak interval, a time interval between zero crossing points, an integral value, and a dispersion value for at least one of an acceleration, an angular velocity, a position, and terrestrial magnetism of the measurement object. With this configuration, for example, the calculation unit 503 can use an angular velocity, a position, and terrestrial magnetism, in addition to an acceleration, and can use a time interval between zero crossing points, an integral value, and a dispersion value, in addition to a peak amplitude and a peak interval.

The calculation unit 503 may calculate, based on the measurement information acquired by the acquisition unit 501, dissimilarity of the first feature amount to the first motion period and similarity of the second feature amount to the second motion period, in the specified boundary period.

For example, the calculation unit 503 refers to a determination pattern of an abnormal motion, specifies a first feature amount used for a determination condition, and calculates a representative value of the first feature amount in the boundary period and a representative value of the first feature amount in the first motion period. The calculation unit 503 calculates the dissimilarity so that the greater a ratio between the representative value of the first feature amount in the boundary period and the representative value of the first feature amount in the first motion period, the greater the dissimilarity. The calculation unit 503 may calculate the dissimilarity so that the closer a ratio between the representative value of the first feature amount in the boundary period and the representative value of the first feature amount in the first motion period is to 1, the smaller the dissimilarity. With this configuration, the calculation unit 503 can calculate one of criteria for evaluating a degree of possibility that an abnormal motion is performed by a living body in the boundary period.

For example, the calculation unit 503 refers to a determination pattern of an abnormal motion, specifies a second feature amount used for a determination condition, and calculates a representative value of the second feature amount in the boundary period and a representative value of the second feature amount in the second motion period. The calculation unit 503 calculates the similarity so that the smaller a ratio between the representative value of the second feature amount in the boundary period and the representative value of the second feature amount in the second motion period, the greater the similarity. With this configuration, the calculation unit 503 can calculate one of criteria for evaluating a degree of possibility that an abnormal motion is performed by a living body in the boundary period.

In addition, the calculation unit 503 may calculate dissimilarity of the first feature amounts between the first half period of the boundary period and the first motion period and similarity of the second feature amounts between the second half period of the boundary period and the second motion period. The first half period and the second half period of the boundary period are respectively a first half portion and a second half portion obtained by dividing the boundary period into two. For example, the first half period and the second half period of the boundary period may be a first half portion and a second half portion obtained by dividing the boundary period into two equal portions.

For example, the calculation unit 503 divides the boundary period into two using a candidate time point at which a ratio of third feature amounts between the first half period and the second half period becomes a maximum, among a plurality of candidate time points for division time points at which the boundary period is divided into the first half period and the second half period. The third feature amount is, for example, a dispersion value of a vertical acceleration. With this configuration, the calculation unit 503 can improve accuracy of calculating one of criteria for evaluating a degree of possibility that an abnormal motion is performed by a living body in the boundary period.

For example, the calculation unit 503 refers to a determination pattern of an abnormal motion, specifies a first feature amount used for a determination condition, and calculates a representative value of the first feature amount in the first half period and a representative value of the first feature amount in the first motion period. The calculation unit 503 calculates the dissimilarity so that the greater a ratio between the representative value of the first feature amount in the first half period and the representative value of the first feature amount in the first motion period, the greater the dissimilarity. With this configuration, the calculation unit 503 can improve accuracy of calculating one of criteria for evaluating a degree of possibility that an abnormal motion is performed by a living body in the boundary period.

For example, the calculation unit 503 refers to a determination pattern of an abnormal motion, specifies a second feature amount used for a determination condition, and calculates a representative value of the second feature amount in the second half period and a representative value of the second feature amount in the second motion period. The calculation unit 503 calculates the similarity so that the smaller a ratio between the representative value of the second feature amount in the second half period and the representative value of the second feature amount in the second motion period, the greater the similarity. With this configuration, the calculation unit 503 can improve accuracy of calculating one of criteria for evaluating a degree of possibility that an abnormal motion is performed by a living body in the boundary period.

For example, the calculation unit 503 may calculate a value indicating a degree of monotonous change in the first feature amount from the first motion period to the first half period in a predetermined direction, and calculate the dissimilarity so that the greater the value, the greater a value of the dissimilarity. The predetermined direction is an increasing direction, for example. With this configuration, the calculation unit 503 can improve accuracy of calculating one of criteria for evaluating a degree of possibility that an abnormal motion is performed by a living body in the boundary period.

For example, the calculation unit 503 may calculate a value indicating a degree of monotonous change in the second feature amount from the second half period to the second motion period in a predetermined direction, and calculate the similarity so that the greater the value, the greater a value of the similarity. The predetermined direction is a decreasing direction, for example. With this configuration, the calculation unit 503 can improve accuracy of calculating one of criteria for evaluating a degree of possibility that an abnormal motion is performed by a living body in the boundary period.

For example, the calculation unit 503 may calculate the dissimilarity so that the greater the number of times the first feature amount exceeds a predetermined value in the boundary period, the greater a value of the dissimilarity. The predetermined value is set based on the representative value of the first feature amount in the first motion period. With this configuration, the calculation unit 503 can improve accuracy of calculating one of criteria for evaluating a degree of possibility that an abnormal motion is performed by a living body in the boundary period.

Then, the calculation unit 503 calculates likelihood of a third motion period. The third motion period is an abnormal motion period. The third motion period is, for example, an abnormal motion such as losing balance in a walking motion. For example, the calculation unit 503 calculates the likelihood so that the greater the dissimilarity, the greater the likelihood, and the greater the similarity, the greater the likelihood. With this configuration, the calculation unit 503 can calculate likelihood corresponding to a degree of possibility that an abnormal motion is performed by a living body in the boundary period.

For example, the calculation unit 503 may calculate the likelihood based on differences between the representative values of the first feature amounts or the second feature amounts in the first motion period and the second motion period. Specifically, the calculation unit 503 calculates the likelihood so that the greater the representative value of the first feature amount in the first motion period than the representative value of the first feature amount in the second motion period, the greater the likelihood. With this configuration, the calculation unit 503 can improve accuracy of calculating likelihood.

For example, the calculation unit 503 may calculate the likelihood based on an index value indicating a degree of possibility that a motion different from a motion performed in the first motion period or a motion performed in the second motion period is performed in a part of the boundary period. Specifically, the calculation unit 503 calculates the likelihood so that the greater the index value, the smaller the likelihood. With this configuration, the calculation unit 503 can improve accuracy of calculating likelihood.

The calculation unit 503 may calculate the likelihood based on the length of the boundary period, for example. Specifically, the calculation unit 503 may calculate the likelihood so that the longer the boundary period, the smaller the likelihood. With this configuration, the calculation unit 503 can improve accuracy of calculating likelihood.

The determination unit 504 determines whether or not the specified boundary period is a third motion period in which a motion is performed by a living body, based on a calculation result by the calculation unit 503. With this configuration, the determination unit 504 can determine whether or not the boundary period is the third motion period by using, as an action feature amount, an angular velocity, a position, and terrestrial magnetism, in addition to or in place of an acceleration.

For example, in a case where a foot is twisted when a balance is lost in a walking motion, an abnormal rotational motion tends to appear in the upper or lower body. In this case, the determination unit 504 can increase determination accuracy by using an amplitude of an angular velocity. In addition, for example, when a balance is lost, a stride may be significantly disturbed. In this case, the determination unit 504 can increase determination accuracy by using a stride for each step calculated from position information.

In addition, the determination unit 504 can determine whether or not the boundary period is the third motion period by using, as a temporal feature amount, a time interval between zero crossing points, an integral value, a dispersion value, or the like, in addition to or in place of a peak amplitude or a peak interval.

For example, in a case where a foot is dragged when a balance is lost in a walking motion, a plurality of peaks of an acceleration may occur per step. In this case, it is difficult for the determination unit 504 to evaluate a landing interval even if a peak interval of an acceleration is used as a temporal feature amount. Therefore, the determination unit 504 can accurately evaluate a landing interval by using, in place of the peak interval of the acceleration, an interval of zero crossing points of a vertical vibration component extracted from the acceleration. In addition, the determination unit 504 can increase determination accuracy based on an evaluation result of a landing interval.

In addition, for example, in a case where the upper body is swayed when a balance is lost in a walking motion, accelerations of the waist between landing time points tend to vary. In this case, it is difficult for the determination unit 504 to evaluate sway of the upper body even if an amplitude of an acceleration peak is used as an action feature amount. Therefore, the determination unit 504 can accurately evaluate the sway of the upper body by using a dispersion value of accelerations between peak points, in place of the amplitude of the acceleration peak. In addition, the determination unit 504 can increase determination accuracy based on an evaluation result of sway of the upper body.

The determination unit 504 may determine whether or not the specified boundary period is a third motion period in which a motion is performed by a living body, based on the dissimilarity and the similarity calculated by the calculation unit 503. For example, in a case where the likelihood is equal to or greater than a threshold, the determination unit 504 determines that the boundary period is the third motion period. Specifically, in a case where the likelihood is equal to or greater than a threshold, the determination unit 504 determines that the boundary period is an abnormal motion period. With this configuration, the determination unit 504 can determine whether or not the boundary period is an abnormal motion period and is included in a motion period of the living body.

The output unit 505 outputs the dissimilarity or the similarity calculated by the calculation unit 503, the likelihood calculated by the calculation unit 503, the determination result by the determination unit 504, and the like. The output unit 505 may output, in association with the boundary period determined to be the third motion period, at least one of an index value indicating a degree of possibility that a motion different from a motion performed in the first motion period and the second motion period is performed in a part of the boundary period, magnitude of a degree of monotonous change in the first feature amount from the first motion period to the first half period of the boundary period in a predetermined direction, magnitude of a degree of monotonous change in the second feature amount from the second half period of the boundary period to the second motion period in a predetermined direction, and the number of times the first feature amount exceeds a predetermined value in the boundary period.

The output is implemented as display on a display, print output to a printer, transmission to an external device by the network I/F 303, or storage to a storage area such as the memory 302 and the recording medium 305, for example. With this configuration, the output unit 505 can provide the dissimilarity or the similarity calculated by the calculation unit 503, the likelihood calculated by the calculation unit 503, the determination result by the determination unit 504, and the like to a patient or a medical person such as a doctor.

The output unit 505 may output information regarding the first motion period and the second motion period detected by the detection unit 502. The output unit 505 outputs, for example, the length of the first motion period and the length of the second motion period. The output unit 505 outputs, for example, a representative value of a predetermined feature amount in the first motion period and a representative value of a predetermined feature amount in the second motion period. The predetermined feature amount is, for example, any one of a peak amplitude, a peak interval, a time interval between zero crossing points, an integral value, and a dispersion value for any one of an acceleration, an angular velocity, a position, and terrestrial magnetism of a living body. The predetermined feature amount is, for example, a peak amplitude of a vertical acceleration, a peak interval of a vertical acceleration, and a dispersion value of a vertical acceleration. With this configuration, the output unit 505 can provide information regarding a motion period to a patient or a medical person such as a doctor.

The output unit 505 may output information regarding the boundary period determined by the determination unit 504 to be the third motion period. The output unit 505 outputs, for example, the length of an abnormal motion period. The output unit 505 outputs, for example, a representative value of the first feature amount and a representative value of the second feature amount, in the abnormal motion period. The output unit 505 outputs, for example, a change rate or monotonous change rate of the first feature amount from the first motion period to the abnormal motion period, and a change rate or monotonous change rate of the second feature amount from the abnormal motion period to the second motion period. The change rate is a ratio between average values of the feature amounts between the two periods, for example. The monotonous change rate is, for example, a slope of an approximate straight line indicating a change in the feature amounts in the two periods. With this configuration, the output unit 505 can provide information regarding an abnormal motion period to a patient or a medical person such as a doctor.

(One Example of Operation Flow of Information Processing Apparatus 100)

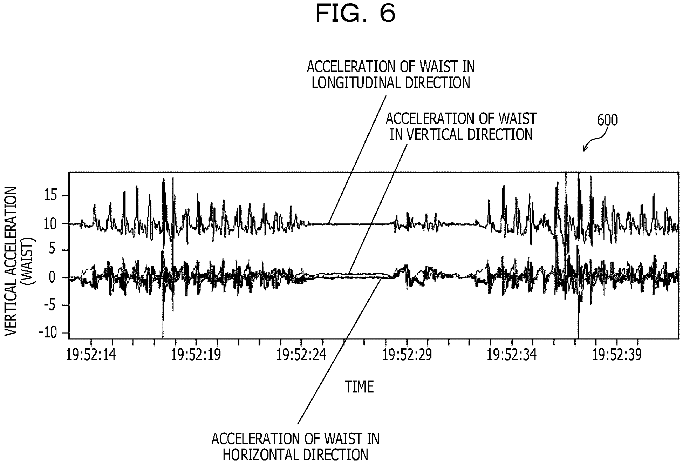

Next, an example of an operation flow of the information processing apparatus 100 will be described with reference to FIGS. 6 to 9.

FIG. 6 is an explanatory diagram illustrating an example of time-series data 600 acquired by the information processing apparatus 100. In the example of FIG. 6, the measuring instrument 101 is mounted to the waist of a patient. The measuring instrument 101 uses the sensor unit 404 to measure a vertical acceleration, a horizontal acceleration, and a longitudinal acceleration. The measuring instrument 101 uses the timer unit 405 to accumulate the measured vertical acceleration, horizontal acceleration, and longitudinal acceleration, in association with time points at which these accelerations are measured.

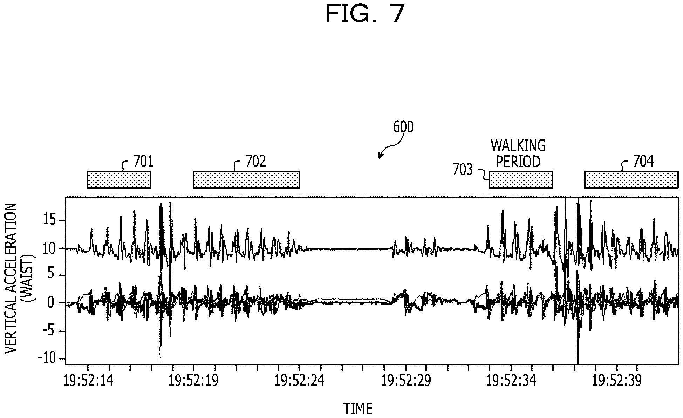

The measuring instrument 101 can create the time-series data 600 illustrated in FIG. 6 as a result of accumulating the vertical acceleration, the horizontal acceleration, and the longitudinal acceleration, which are measured for a certain period in association with the time points at which these accelerations are measured. After creating the time-series data 600 illustrated in FIG. 6, the measuring instrument 101 transmits the time-series data 600 to the information processing apparatus 100. The information processing apparatus 100 receives the time-series data 600 illustrated in FIG. 6 from the measuring instrument 101. Next, description of FIG. 7 will be made.

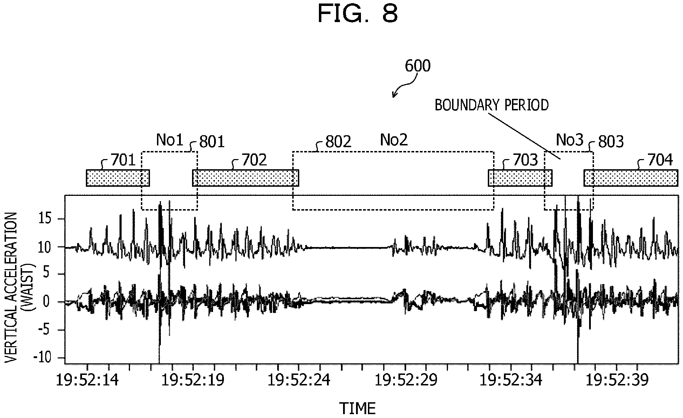

FIG. 7 is an explanatory diagram illustrating an example in which the information processing apparatus 100 detects a walking period. In FIG. 7, the information processing apparatus 100 detects, for the vertical acceleration, the horizontal acceleration, or the longitudinal acceleration in the time-series data 600 illustrated in FIG. 6, walking periods 701 to 704 in which a characteristic pattern in which a zero crossing point of a vertical vibration component extracted from these accelerations periodically appears. For detection of a walking period, for example, Japanese Laid-open Patent Publication No. 2008-171347 can be referred to. Next, description of FIG. 8 will be made.

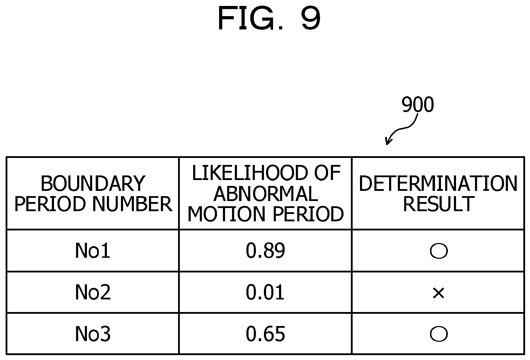

FIG. 8 is an explanatory diagram illustrating an example in which the information processing apparatus 100 specifies a boundary period. In FIG. 8, the information processing apparatus 100 specifies boundary periods 801 to 803 each of which is sandwiched between two of the specified walking periods 701 to 704 as periods that may be abnormal motion periods. The information processing apparatus 100 assigns boundary period numbers "No1 to No3" to the boundary periods 801 to 803, respectively. Next, description of FIG. 9 will be made.

FIG. 9 is an explanatory diagram illustrating an example in which the information processing apparatus 100 determines whether or not the boundary period is an abnormal motion period. In FIG. 9, the information processing apparatus 100 calculates, for each of the specified boundary periods 801 to 803, likelihood of an abnormal motion period in which an abnormal motion such as losing balance in a walking motion is performed, and determines, based on the likelihood, whether or not the boundary periods 801 to 803 are abnormal motion periods.

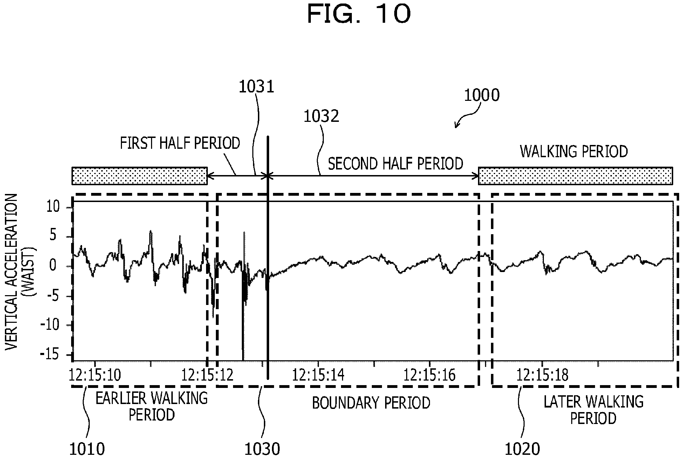

Specific examples for calculating the likelihood will be described later with reference to FIGS. 10 to 33. The information processing apparatus 100 stores the calculated likelihood and a determination result in a determination information table 900 illustrated in FIG. 9.

The determination information table 900 is implemented by a storage area such as the memory 302 or the recording medium 305 of the information processing apparatus 100 illustrated in FIG. 3, for example. The determination information table 900 has fields of a boundary period number, likelihood of an abnormal motion period, and a determination result. In the determination information table 900, determination information is stored as a record by setting information in each field for each boundary period.

In the field of a boundary period number, a boundary period number assigned to a boundary period to be determined whether or not the boundary period is an abnormal motion period is set. In the field of likelihood of an abnormal motion period, likelihood of an abnormal motion period in which any one of a plurality of abnormal motions is performed, which is calculated for the boundary period, is set. In the field of a determination result, whether or not the likelihood is equal to or greater than a threshold is set. For example, a determination result "○" indicates that the likelihood is equal to or greater than a threshold, and indicates that the boundary period is determined to be an abnormal motion period.

In the example of FIG. 9, the information processing apparatus 100 calculates "0.89" as likelihood of an abnormal motion period of the boundary period 801. The information processing apparatus 100 compares the calculated likelihood "0.89" with a threshold "0.5". Since the likelihood is equal to or greater than the threshold, the information processing apparatus 100 determines that the boundary period 801 is an abnormal motion period. Therefore, the information processing apparatus 100 creates, for the boundary period 801, a record in which the boundary period number "No1", the likelihood of an abnormal motion period "0.89", and the determination result "○" are associated with each other, and stores the record in the determination information table 900.

The information processing apparatus 100 may calculate the likelihood of an abnormal motion period in which each of a plurality of abnormal motions is performed, and determine which one of the plurality of abnormal motions is performed in the boundary period 801 as the abnormal motion period. With this configuration, the information processing apparatus 100 can improve accuracy of specifying an abnormal motion period in which each abnormal motion is performed.

Here, for example, in a case where a patient performs an abnormal motion such as losing balance in a standing position maintenance motion, it is considered that an abrupt change occurs in an acceleration in any direction of the waist of the patient. Therefore, in the case of determining whether or not the boundary period 801 is an abnormal motion period in which an abnormal motion such as losing balance in a standing position maintenance motion is performed, the information processing apparatus 100 preferably uses, as a first feature amount, a dispersion value of a synthetic acceleration of a living body. In addition, in this case, the information processing apparatus 100 preferably uses, as a second feature amount, a dispersion value of the synthetic acceleration of the living body.

In addition, for example, in a case where a patient performs an abnormal motion such as losing balance in a stair climbing motion, it is considered that an abrupt change occurs in a vertical acceleration of the waist of the patient. Therefore, in the case of determining whether or not the boundary period 801 is an abnormal motion period in which an abnormal motion such as losing balance in a stair climbing motion is performed, the information processing apparatus 100 preferably uses, as a first feature amount, a peak amplitude of an acceleration in a vertical direction of a living body. In addition, in this case, the information processing apparatus 100 preferably uses, as a second feature amount, a peak interval of the acceleration in the vertical direction of the living body.

In addition, for example, in a case where a patient performs an abnormal motion such as losing balance in a walking motion, it is considered that an abrupt change occurs in a vertical acceleration of the waist of the patient, or an abrupt change occurs in a longitudinal acceleration of the waist of the patient. Therefore, in the case of determining whether or not the boundary period 801 is an abnormal motion period in which an abnormal motion such as losing balance in a walking motion is performed, the information processing apparatus 100 may use, as a first feature amount, a peak amplitude of an acceleration in a longitudinal direction of a living body. In addition, in this case, the information processing apparatus 100 may use, as a second feature amount, a peak interval of the acceleration in the longitudinal direction of the living body.

In addition, for example, in a case where a patient performs an abnormal motion such as losing balance in a cycling motion, it is considered that an abrupt change occurs in a horizontal acceleration of the waist of the patient. Therefore, in the case of determining whether or not the boundary period 801 is an abnormal motion period in which an abnormal motion such as losing balance in a cycling motion is performed, the information processing apparatus 100 preferably uses, as a first feature amount, a peak amplitude of an acceleration in a horizontal direction of a living body. The information processing apparatus 100 preferably uses, as a second feature amount, a dispersion value of the acceleration in the horizontal direction of the living body.

When determining whether or not the boundary period is an abnormal motion period in which each of a plurality of abnormal motions is performed, the information processing apparatus 100 can compare likelihood of an abnormal motion period in which each abnormal motion is performed. Therefore, the information processing apparatus 100 can determine which one of a plurality of abnormal motions is performed in an abnormal motion period.

The information processing apparatus 100 may determine, based on motions performed in motion periods before and after the boundary period 801, whether or not the boundary period 801 is an abnormal motion period in which an abnormal motion related to the motions is performed. With this configuration, the information processing apparatus 100 can reduce a processing amount as compared with a case of calculating the likelihood of an abnormal motion period in which each of a plurality of abnormal motions is performed.

(Specific Example in which Information Processing Apparatus 100 Calculates Likelihood)

Next, a specific example in which the information processing apparatus 100 calculates likelihood will be described with reference to FIGS. 10 to 26.

FIG. 10 is an explanatory diagram illustrating an example of a boundary period for which likelihood is calculated. A graph 1000 of FIG. 10 shows, for each time, a vertical acceleration for that time, and corresponds to time-series data of a vertical acceleration. A horizontal axis of the graph 1000 of FIG. 10 is time. A vertical axis of the graph 1000 of FIG. 10 is a vertical acceleration. A vertical acceleration having a positive value is an acceleration in an upward direction, and a vertical acceleration having a negative value is an acceleration in a downward direction.

An earlier walking period 1010 is detected on an earlier side of the graph 1000 of FIG. 10. Similarly, a later walking period 1020 is detected on a later side of the graph 1000 of FIG. 10. In addition, a boundary period 1030 sandwiched between the detected earlier walking period 1010 and later walking period 1020 is specified in the center of the graph 1000 of FIG. 10. The information processing apparatus 100 selects the boundary period 1030 as an object for calculating likelihood.

Here, there is a property that, if a patient performs an abnormal motion in the boundary period 1030, any part of the patient's body tends to move faster on an earlier side of the boundary period 1030, and a representative value of a peak amplitude of a vertical acceleration of the part tends to increase. According to this property, dissimilarity of a first feature amounts between the boundary period 1030 and the earlier walking period 1010 can be one of criteria for evaluating a degree of possibility that an abnormal motion is performed by the patient. In particular, dissimilarity of the first feature amounts between a first half period 1031 of the boundary period 1030 and the earlier walking period 1010 can be one of criteria for more accurately evaluating a degree of possibility that an abnormal motion is performed by the patient.

In addition, there is a property that, if the patient performs an abnormal motion in the boundary period 1030, the movement tends to gradually return to a movement before the patient performs the abnormal motion, on a later side of the boundary period 1030, and any part of the patient's body tends to move slow. According to this property, similarity of a second feature amounts between the boundary period 1030 and the later walking period 1020 can be one of criteria for evaluating a degree of possibility that an abnormal motion is performed by the patient. In particular, similarity of the second feature amounts between a second half period 1032 of the boundary period 1030 and the later walking period 1020 can be one of criteria for more accurately evaluating a degree of possibility that an abnormal motion is performed by the patient.

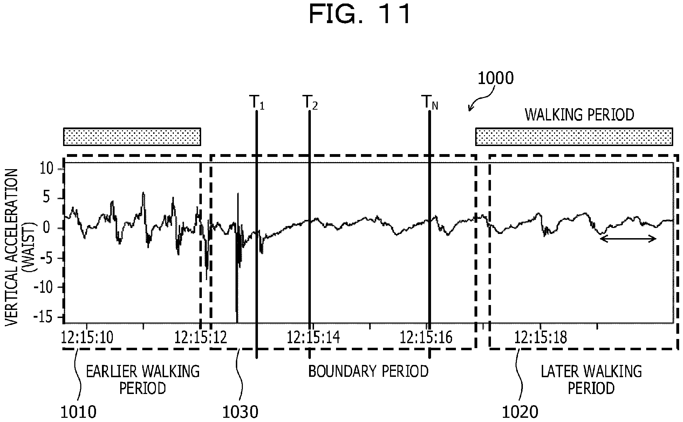

Therefore, the information processing apparatus 100 preferably divides the boundary period 1030 into the first half period 1031 and the second half period 1032 before calculating the dissimilarity of the first feature amounts and the similarity of the second feature amounts. Specific examples for dividing the boundary period 1030 will be described later with reference to FIG. 11.

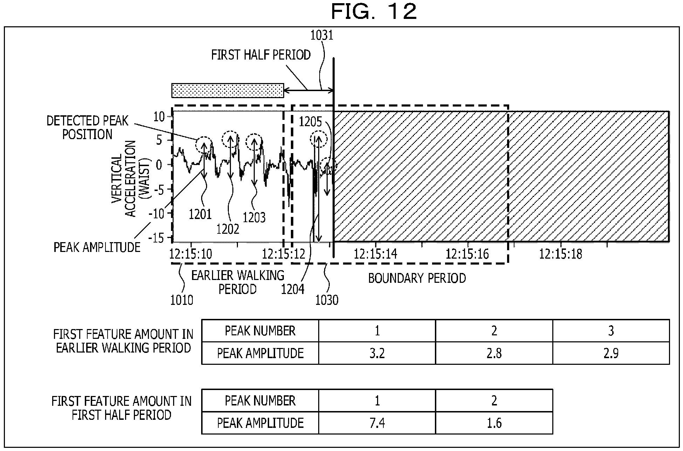

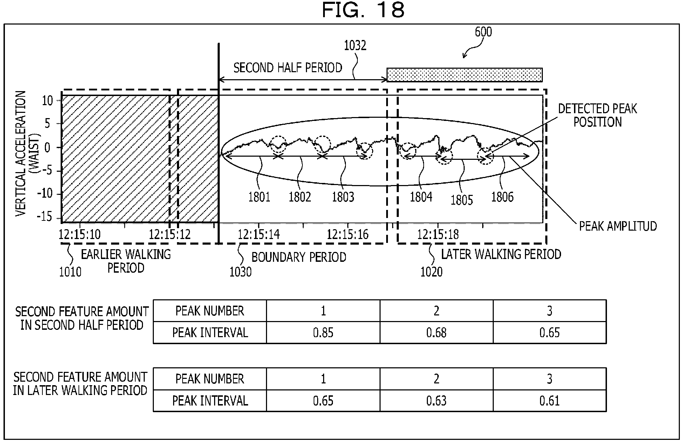

Then, the information processing apparatus 100 calculates the dissimilarity of the first feature amounts between the first half period 1031 and the earlier walking period 1010 and the similarity of the second feature amounts between the second half period 1032 and the later walking period 1020. Specific examples for calculating the dissimilarity of the first feature amounts will be described later with reference to FIGS. 12 to 17. Specific examples for calculating the similarity of the second feature amounts will be described later with reference to FIGS. 18 to 21.

With this configuration, the information processing apparatus 100 can set the dissimilarity of the first feature amounts and the similarity of the second feature amounts, which are to be calculated, as criteria for more accurately evaluating a degree of possibility that an abnormal motion is performed by the patient in the boundary period 1030. Therefore, the information processing apparatus 100 can improve accuracy of calculating likelihood based on the criteria.

FIG. 11 is an explanatory diagram illustrating a specific example in which the information processing apparatus 100 divides the boundary period 1030. In FIG. 11, the information processing apparatus 100 prepares, depending on the length of the boundary period 1030, N candidate time points $T_1$, $T_2$, ..., $T_n$ for division time points at which the boundary period 1030 is divided into the first half period 1031 and the second half period 1032.

The information processing apparatus 100 selects a candidate time point at which a ratio of third feature amounts between the first half period 1031 and the second half period 1032 becomes a maximum, among the prepared N candidate time points $T_1$, $T_2$, ..., $T_n$. Specifically, the information processing apparatus 100 selects a candidate time point at which a ratio of dispersion values of vertical accelerations between the first half period 1031 and the second half period 1032 becomes a maximum.

Then, the information processing apparatus 100 divides the boundary period 1030 into the first half period 1031 and the second half period 1032 at the selected candidate time point. With this configuration, the information processing apparatus 100 can divide the boundary period 1030 so that the dissimilarity of the first feature amounts and the similarity of the second feature amounts are set as criteria for more accurately evaluating a degree of possibility that an abnormal motion is performed by the patient in the boundary period 1030.

<Specific Examples of Calculating Dissimilarity of First Feature Amounts>

FIGS. 12 to 14 are explanatory diagrams illustrating a first example of calculating dissimilarity of first feature amounts. Here, the information processing apparatus 100 refers to a determination pattern of an abnormal motion such as losing balance in a walking motion, and uses a peak amplitude of a vertical acceleration as a first feature amount.

In FIG. 12, the information processing apparatus 100 detects peaks of a vertical acceleration in the earlier walking period 1010 and calculates peak amplitudes 1201 to 1203 of the vertical acceleration in the earlier walking period 1010. The peak is a time point at which the vertical acceleration becomes a local maximum or a local minimum. Specifically, the peak amplitudes 1201 to 1203 are assigned peak numbers 1 to 3, and have values of 3.2, 2.8, and 2.9, respectively.

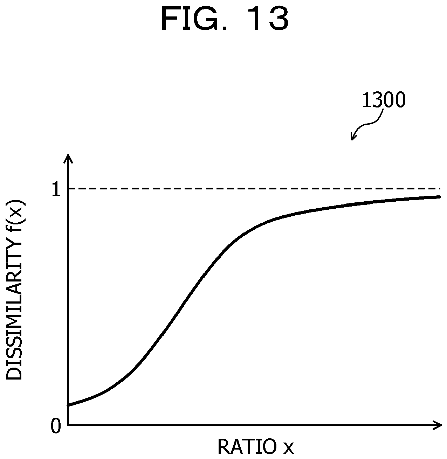

In addition, the information processing apparatus 100 detects peaks of a vertical acceleration in the first half period 1031 and calculates peak amplitudes 1204 and 1205 of the vertical acceleration in the first half period 1031. Specifically, the peak amplitudes 1204 and 1205 are assigned peak numbers 1 and 2, and have values of 7.4 and 1.6, respectively. With this configuration, the information processing apparatus 100 can prepare for calculating the dissimilarity of the first feature amounts. Next, description of FIG. 13 will be made.

A graph 1300 of FIG. 13 shows dissimilarity of peak amplitudes with respect to a ratio between a maximum value of the peak amplitudes 1201 to 1203 of the vertical acceleration in the earlier walking period 1010 and a maximum value of the peak amplitudes 1204 and 1205 of the vertical acceleration in the first half period 1031. The graph 1300 of FIG. 13 is represented by the following Formula (1).

[Mathematical Formula 1]

$$f(x) = \frac{1}{1 + exp(\alpha_1 x - \alpha_2)} \quad ...(1)$$

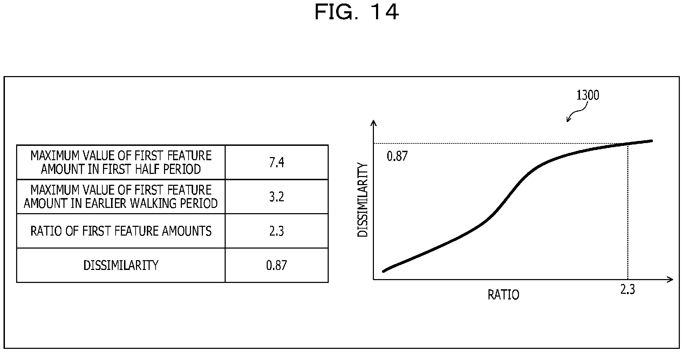

Here, x is a ratio of representative values of the first feature amounts between the earlier walking period 1010 and the first half period 1031. For example, x is a ratio of maximum values of peak amplitudes between the earlier walking period 1010 and the first half period 1031. f(x) is dissimilarity of the first feature amounts between the earlier walking period 1010 and the first half period 1031. $\alpha_1$ and $\alpha_2$ are parameters for deciding shapes of functions. $\alpha_1$ is negative. The information processing apparatus 100 can calculate the dissimilarity of the peak amplitudes by using the graph 1300 of FIG. 13. Next, description of FIG. 14 will be made.

In FIG. 14, the information processing apparatus 100 calculates a ratio 2.3 between a maximum value 3.2 of the peak amplitudes of the vertical acceleration in the earlier walking period 1010 and a maximum value 7.4 of the peak amplitudes of the vertical acceleration in the first half period 1031, and substitutes the ratio into the above-described Formula (1). The information processing apparatus 100 calculates dissimilarity 0.87 by the above-described Formula (1) and stores the value. With this configuration, the information processing apparatus 100 can acquire one of criteria for evaluating a degree of possibility that an abnormal motion is performed by the patient in the boundary period 1030, and use the criterion when calculating likelihood.

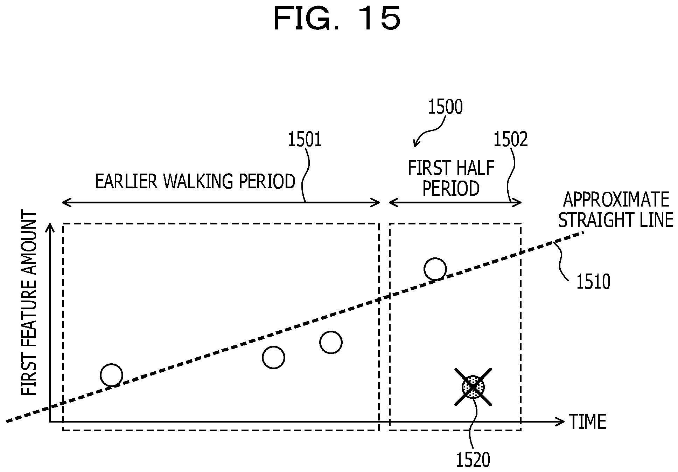

FIG. 15 is an explanatory diagram illustrating a second example of calculating dissimilarity of the first feature amounts. A graph 1500 of FIG. 15 shows, for each time, a first feature amount of that time. A horizontal axis of the graph 1500 of FIG. 15 is time. A vertical axis of the graph 1500 of FIG. 15 is the first feature amount, for example, a peak amplitude of a vertical acceleration. On the graph 1500 of FIG. 15, the first feature amount at each time is indicated by using "○".

Here, if a patient performs an abnormal motion in a first half period of a boundary period, a peak amplitude of a vertical acceleration of any part of a patient's body tends to increase from an earlier walking period to the first half period. For this reason, there is a property that peak amplitudes of vertical accelerations of the earlier walking period and the first half period are unlikely to be similar to each other. According to this property, dissimilarity of the first feature amounts between the first half period and the earlier walking period can also be expressed by a degree of increase of a peak amplitude of a vertical acceleration from the earlier walking period to the first half period.

Therefore, the information processing apparatus 100 calculates a value indicating a degree of monotonous increase of the first feature amount from an earlier walking period 1501 to a first half period 1502, as dissimilarity of the first feature amounts between the earlier walking period 1501 and the first half period 1502. The information processing apparatus 100 calculates, for example, a slope of an approximate straight line 1510 of a peak amplitude of a vertical acceleration from the earlier walking period 1501 to the first half period 1502, as dissimilarity of peak amplitudes of vertical accelerations between the earlier walking period 1501 and the first half period 1502. With this configuration, the information processing apparatus 100 can acquire one of criteria for evaluating a degree of possibility that an abnormal motion is performed by a patient in a boundary period, and use the criterion when calculating likelihood.

Here, in the first half period of the boundary period, the peak amplitude of the vertical acceleration of any part of the patient's body increases until immediately after the patient starts an abnormal motion. However, there is a possibility that the peak amplitude decreases after immediately after the patient starts the abnormal motion. Accordingly, there is a case where the dissimilarity of the first feature amounts between the first half period and the earlier walking period is preferably expressed by a degree of increase of a peak amplitude of a vertical acceleration from the earlier walking period to a time point at which the peak amplitude of the vertical acceleration becomes a maximum in the first half period.

For this reason, it may be possible that the information processing apparatus 100 does not use a peak amplitude after the peak amplitude of the vertical acceleration becomes a maximum in the first half period 1502, when obtaining the approximate straight line 1510. The information processing apparatus 100 does not use a peak amplitude 1520, for example. With this configuration, the information processing apparatus 100 can calculate one of criteria for more accurately evaluating a degree of possibility that an abnormal motion is performed by a patient in a boundary period.

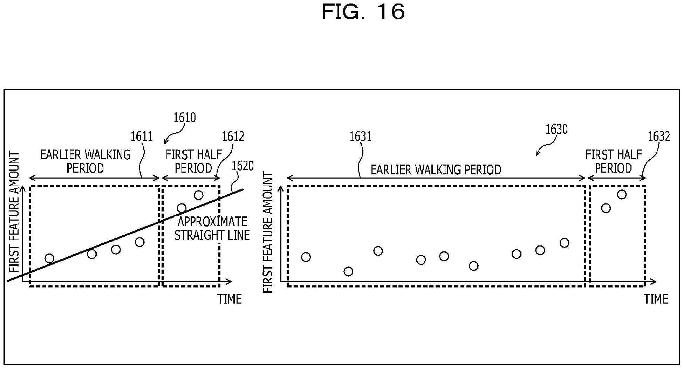

FIG. 16 is an explanatory diagram illustrating an example of selectively using the first example and the second example of calculating the dissimilarity of the first feature amounts. For example, the information processing apparatus 100 may selectively use the first example and the second example described above of calculating the dissimilarity of the first feature amounts, depending on the length of an earlier walking period.

Specifically, the information processing apparatus 100 uses either the first example or the second example depending on an estimated length of the earlier walking period, which is set by a patient or a medical person such as a doctor. In addition, specifically, the information processing apparatus 100 may selectively use the first example and the second example depending on a detected length of the earlier walking period.

Here, as in a graph 1610 of FIG. 16, the length of an earlier walking period 1611 may be relatively short as compared with that of a first half period 1612. In this case, it is considered that an influence of a start of an abnormal motion tends to appear also in the first feature amount in the earlier walking period 1611, and the first feature amount tends to monotonously increase. Therefore, in a case where the length of the earlier walking period 1611 is relatively short, the information processing apparatus 100 preferably uses the second example described above, and preferably calculates a slope of an approximate straight line 1620 as dissimilarity. With this configuration, the information processing apparatus 100 can more accurately calculate one of criteria for evaluating a degree of possibility that an abnormal motion is performed by a patient.

On the other hand, as in a graph 1630 of FIG. 16, the length of an earlier walking period 1631 may be relatively long as compared with that of a first half period 1632. In this case, it is considered that an influence of a start of an abnormal motion tends not to appear in the first feature amount in the earlier walking period 1631, and the first feature amount tends not to monotonously increase. Therefore, in a case where the length of the earlier walking period 1631 is relatively long, the information processing apparatus 100 preferably uses the first example described above. With this configuration, the information processing apparatus 100 can more accurately calculate one of criteria for evaluating a degree of possibility that an abnormal motion is performed by a patient.

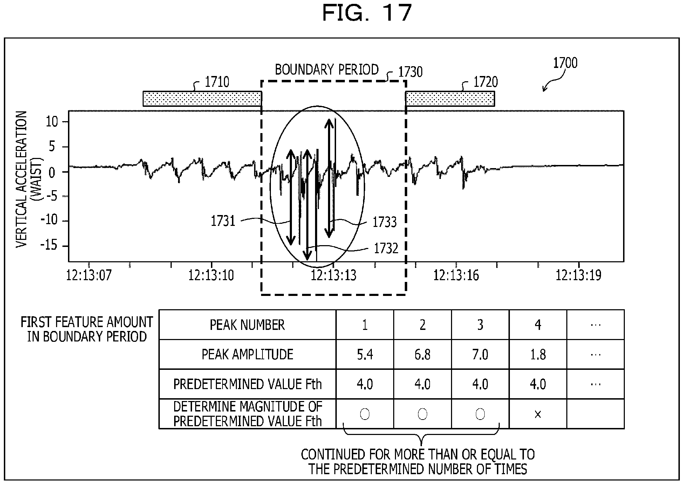

FIG. 17 is an explanatory diagram illustrating a third example of calculating dissimilarity of the first feature amounts. In the example of FIG. 17, a graph different from the graph 1000 of FIG. 10 is used. A graph 1700 of FIG. 17 shows, for each time, a vertical acceleration for that time, and corresponds to time-series data of a vertical acceleration. A horizontal axis of the graph 1700 of FIG. 17 is time. A vertical axis of the graph 1700 of FIG. 17 is a vertical acceleration.

An earlier walking period 1710 is detected on an earlier side of the graph 1700 of FIG. 17. Similarly, a later walking period 1720 is detected on a later side of the graph 1700 of FIG. 17. In addition, a boundary period 1730 sandwiched between the detected earlier walking period 1710 and later walking period 1720 is specified in the center of the graph 1700 of FIG. 17. The information processing apparatus 100 selects the boundary period 1730 as an object for calculating likelihood.

Here, there is a property that, if a patient performs an abnormal motion in the boundary period 1730, a peak amplitude of a vertical acceleration in the boundary period 1730 tends to be greater than a peak amplitude of a vertical acceleration in the earlier walking period 1710. According to this property, dissimilarity of the first feature amounts between the boundary period 1730 and the earlier walking period 1710 can be expressed also by the number of peak amplitudes that are equal to or greater than a threshold based on the peak amplitude of the vertical acceleration in the earlier walking period 1710, among peak amplitudes of the vertical acceleration in the boundary period 1730.

Therefore, the information processing apparatus 100 calculates an average value 4.0 of peak amplitudes of the vertical acceleration in the earlier walking period 1710 as a predetermined value $F_{th}$ serving as a threshold. The information processing apparatus 100 calculates peak amplitudes 1731 to 1733 of the vertical acceleration in the boundary period 1730, and the like. Specifically, the peak amplitudes 1731 to 1733, and the like are assigned peak numbers 1 to 3, and the like, and have values of 5.4, 6.8, 7.0, and the like, respectively.

The information processing apparatus 100 calculates the number of times peak amplitudes equal to or greater than the predetermined value $F_{th}$ are continued, among the calculated peak amplitudes of the vertical acceleration in the boundary period 1730. The information processing apparatus 100 calculates and stores the number of consecutive times or a value based on the number of consecutive times, as dissimilarity of peak amplitudes of the vertical accelerations between the earlier walking period 1710 and the boundary period 1730. With this configuration, the information processing apparatus 100 can acquire one of criteria for evaluating a degree of possibility that an abnormal motion is performed by a patient in the boundary period 1730.

The information processing apparatus 100 may calculate the dissimilarity of the first feature amounts by combining at least two or more of the above-described first example, second example, and third example of calculating the dissimilarity of the first feature amounts.

<Specific Examples of Calculating Similarity of Second Feature Amounts>

FIGS. 18 to 20 are explanatory diagrams illustrating a first example of calculating similarity of second feature amounts. Here, the information processing apparatus 100 refers to a determination pattern of an abnormal motion such as losing balance in a walking motion, and uses a peak interval of a vertical acceleration as a second feature amount.

In FIG. 18, the information processing apparatus 100 calculates peak intervals 1801 to 1803 of the vertical acceleration in the second half period 1032. Specifically, the peak intervals 1801 to 1803 are assigned peak numbers 1 to 3, and have values of 0.85, 0.68, and 0.65, respectively.

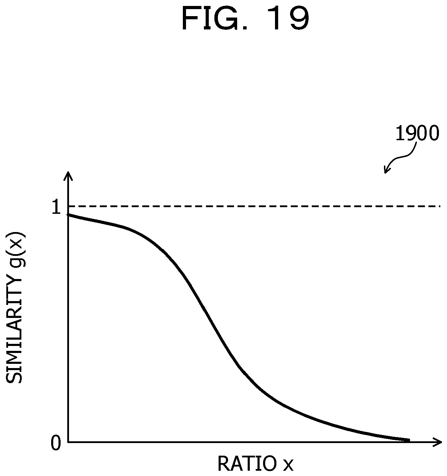

In addition, the information processing apparatus 100 calculates peak intervals 1804 to 1806 of the vertical acceleration in the later walking period 1020. Specifically, the peak intervals 1804 to 1806 are assigned peak numbers 1 to 3, and have values of 0.65, 0.63, and 0.61, respectively. With this configuration, the information processing apparatus 100 can prepare for calculating the similarity of the second feature amounts. Next, description of FIG. 19 will be made.

A graph 1900 of FIG. 19 shows similarity of peak intervals with respect to a ratio between a maximum value of the peak intervals 1801 to 1803 of the vertical acceleration in the second half period 1032 and a maximum value of the peak intervals 1804 to 1806 of the vertical acceleration in the later walking period 1020. The graph 1900 of FIG. 19 is represented by the following Formula (2).

[Mathematical Formula 2]

$$g(x) = \frac{1}{1 + exp(\beta_1 x - \beta_2)} \quad \cdots (2)$$

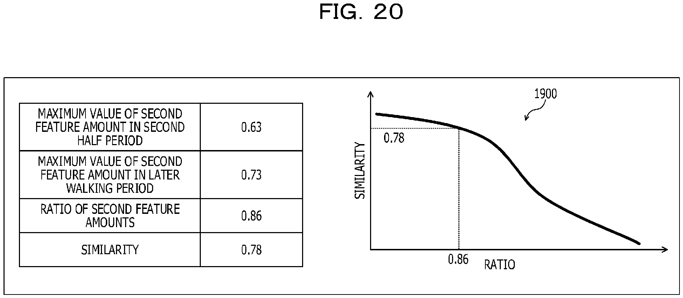

Here, x is a ratio of representative values of the second feature amounts between the second half period 1032 and the later walking period 1020. For example, x is a ratio of maximum values of peak intervals between the second half period 1032 and the later walking period 1020. g(x) is similarity of the second feature amounts between the second half period 1032 and the later walking period 1020. $\beta_1$ and $\beta_2$ are parameters for deciding shapes of functions. $\beta_1$ is positive. The information processing apparatus 100 can calculate similarity of the peak intervals by using the graph 1900 of FIG. 19. Next, description of FIG. 20 will be made.

In FIG. 20, the information processing apparatus 100 calculates a ratio 0.86 between a maximum value 0.63 of the peak intervals of the vertical acceleration in the second half period 1032 and a maximum value 0.73 of the peak intervals of the vertical acceleration in the later walking period 1020, and substitutes the ratio into the above-described Formula (2). The information processing apparatus 100 calculates similarity 0.78 by the above-described Formula (2) and stores the value. With this configuration, the information processing apparatus 100 can acquire one of criteria for evaluating a degree of possibility that an abnormal motion is performed by the patient in the boundary period 1030, and use the criterion when calculating likelihood.

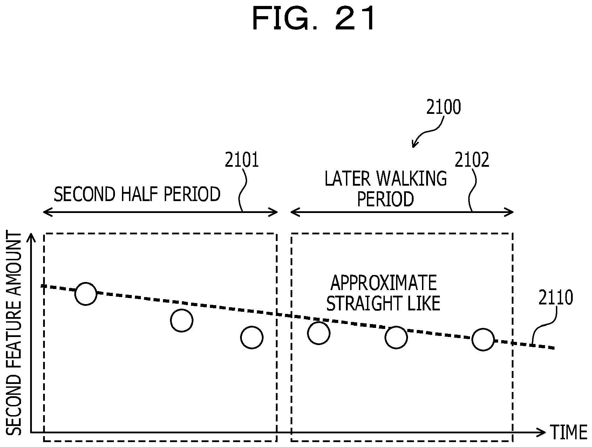

FIG. 21 is an explanatory diagram illustrating a second example of calculating similarity of the second feature amounts. A graph 2100 of FIG. 21 shows, for each time, a second feature amount of that time. A horizontal axis of the graph 2100 of FIG. 21 is time. A vertical axis of the graph 2100 of FIG. 21 is the second feature amount, for example, a peak interval of a vertical acceleration. On the graph 2100 of FIG. 21, the second feature amount at each time is indicated by using "O".

Here, there is a property that, if a patient gradually recovers from an abnormal motion in a second half period of a boundary period, a peak interval of a vertical acceleration of any part of a patient's body tends to be gradually stabilized from a large value to a small value from the second half period to a later walking period. For this reason, there is a property that peak intervals of vertical accelerations of the second half period and the later walking period are likely to be similar to each other. According to this property, similarity of the second feature amounts between the second half period and the later walking period can also be expressed by a degree of decrease of a peak interval of a vertical acceleration from the second half period to the later walking period.

Therefore, the information processing apparatus 100 calculates a value indicating a degree of monotonous decrease of the second feature amount from a second half period 2101 to a later walking period 2102, as similarity of the second feature amounts between the second half period 2101 and the later walking period 2102. The information processing apparatus 100 calculates, for example, a slope of an approximate straight line 2110 of a peak interval of a vertical acceleration from the second half period 2101 to the later walking period 2102, as similarity of peak intervals of vertical accelerations between the later walking period 2102 and the second half period 2101. With this configuration, the information processing apparatus 100 can acquire one of criteria for evaluating a degree of possibility that an abnormal motion is performed by a patient in a boundary period, and use the criterion when calculating likelihood.

<Specific Examples of Calculating Likelihood>

Next, a first example of calculating likelihood will be described in which the information processing apparatus 100 calculates, for the boundary period 1030, likelihood of an abnormal motion period in which an abnormal motion such as losing balance in a walking motion is performed, based on the calculated dissimilarity of the first feature amounts and the calculated similarity of the second feature amounts.

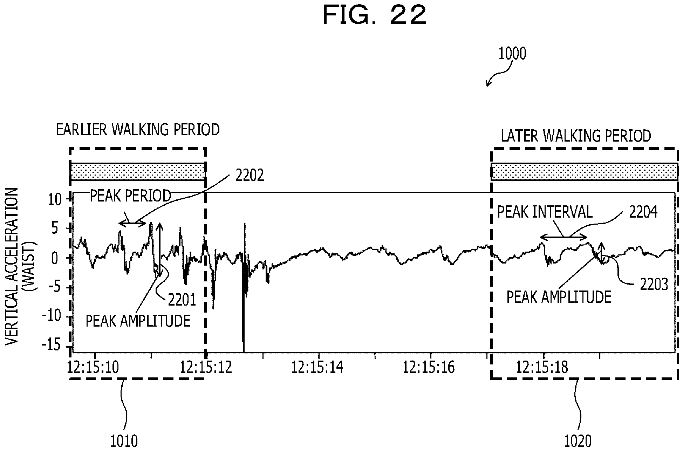

For example, the information processing apparatus 100 calculates, as the likelihood, a value obtained by multiplying dissimilarity $D_1$ of the first feature amounts by similarity $D_2$ of the second feature amounts. With this configuration, the information processing apparatus 100 can calculate the likelihood so that the greater the dissimilarity $D_1$, the greater the likelihood, and the greater the similarity $D_2$, the greater the likelihood. In addition, the information processing apparatus 100 can evaluate the likelihood of an abnormal motion period. Next, description of FIG. 22 will be made.

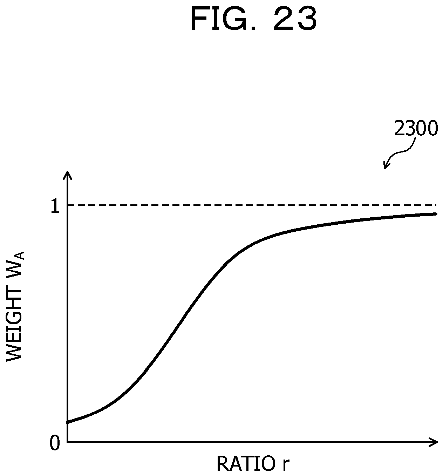

FIGS. 22 and 23 are explanatory diagrams illustrating a second example of calculating likelihood. Here, if a patient has performed an abnormal motion in the boundary period 1030, the patient tends to perform a motion more carefully in the later walking period 1020 than in the earlier walking period 1010. With this reason, a difference between representative values of the first feature amounts or the second feature amounts of the earlier walking period 1010 and the later walking period 1020 can be one of criteria for evaluating a degree of possibility that an abnormal motion is performed in the boundary period 1030 by the patient.

Here, specifically, it is conceivable that, when the patient performs a motion carefully, a movement of the patient becomes slow and a stride of the patient becomes large. For this reason, specifically, it is conceivable that, when the patient performs a motion carefully, a peak amplitude becomes small and a peak interval becomes large.

In FIG. 22, the information processing apparatus 100 calculates a peak amplitude 2201 and a peak interval 2202 in the earlier walking period 1010. In addition, the information processing apparatus 100 calculates a peak amplitude 2203 and a peak interval 2204 in the later walking period 1020.

The information processing apparatus 100 calculates a difference between the peak amplitude 2201 in the earlier walking period 1010 and the peak amplitude 2203 in the later walking period 1020. In addition, the information processing apparatus 100 calculates a difference between the peak interval 2202 in the earlier walking period 1010 and the peak interval 2204 in the later walking period 1020. Then, the information processing apparatus 100 calculates, based on the calculated differences, the likelihood by the following Formula (3).
[Mathematical Formula 3]

$$L = w_A \times D_1 \times D_2 \quad ...(3)$$

Here, $w_A$ is the weight set based on the calculated differences. A specific example of setting $w_A$ will be described later with reference to FIG. 23. $D_1$ is dissimilarity of the first feature amounts. $D_2$ is similarity of the second feature amounts. Next, description of FIG. 23 will be made.

A graph 2300 of FIG. 23 shows the weight with respect to the calculated differences. The graph 2300 of FIG. 23 is represented by the following Formula (4).
[Mathematical Formula 4]

$$w_A = \frac{1}{1 + exp(\lambda_1 r - \lambda_2)} \quad ...(4)$$

Here, r is a calculated ratio. $\lambda_1$, and $\lambda_2$ are parameters for deciding shapes of functions. $\lambda_1$ is negative. The information processing apparatus 100 sets a weight

$w_A$ corresponding to a calculated ratio by using the graph 2300 of FIG. 23, and calculates the likelihood. With this configuration, the information processing apparatus 100 can improve accuracy of calculating likelihood.

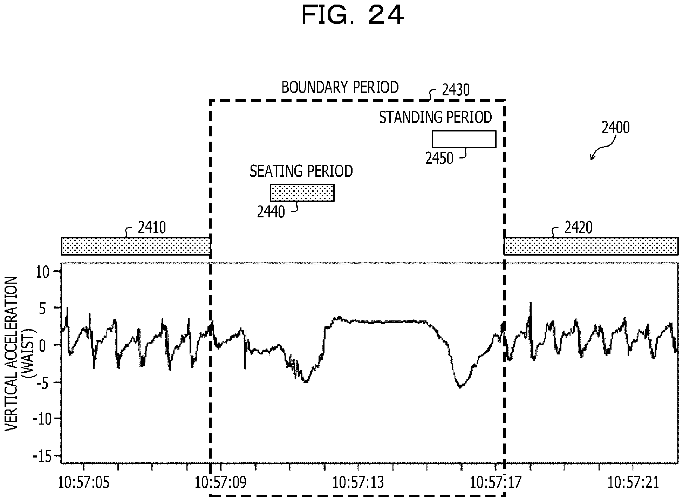

FIG. 24 is an explanatory diagram illustrating a third example of calculating the likelihood. In the example of FIG. 24, a graph different from the graph 1000 of FIG. 10 is used. A graph 2400 of FIG. 24 shows, for each time, a vertical acceleration for that time, and corresponds to time-series data of a vertical acceleration. A horizontal axis of the graph 2400 of FIG. 24 is time. A vertical axis of the graph 2400 of FIG. 24 is a vertical acceleration.

An earlier walking period 2410 is detected on an earlier side of the graph 2400 of FIG. 24. Similarly, a later walking period 2420 is detected on a later side of the graph 2400 of FIG. 24. In addition, a boundary period 2430 sandwiched between the detected earlier walking period 2410 and later walking period 2420 is specified in the center of the graph 2400 of FIG. 24.

It is assumed that the information processing apparatus 100 detects, in addition to a walking period, a seating period 2440 in which a patient is seated, a standing period 2450 in which a patient is standing, and the like. The information processing apparatus 100 detects, for example, the seating period 2440, the standing period 2450, and the like based on whether or not a predetermined feature amount in a certain period satisfies a condition corresponding to seating and standing of a patient.

In the example of FIG. 24, the detected seating period 2440, standing period 2450, and the like are included in the boundary period 2430. For detection of a seating period and a standing period, for example, SVM-based posture identification with a single waist-located triaxial accelerometer, which is a non-patent document, can be referred to.

Here, there is a property that, in the boundary period 2430 sandwiched between the earlier walking period 2410 and the later walking period 2420, in a case where a motion other than a walking motion is performed, there is a low possibility that an abnormal motion related to a walking motion is performed. According to this property, in a case where the boundary period 2430 includes the seating period 2440, the standing period 2450, and the like, it is considered to be preferable to decrease the likelihood.

Therefore, since the detected seating period 2440, standing period 2450, and the like are included in the boundary period 2430, the information processing apparatus 100 calculates the likelihood so as to reduce the likelihood. With this configuration, the information processing apparatus 100 can improve accuracy of calculating likelihood.

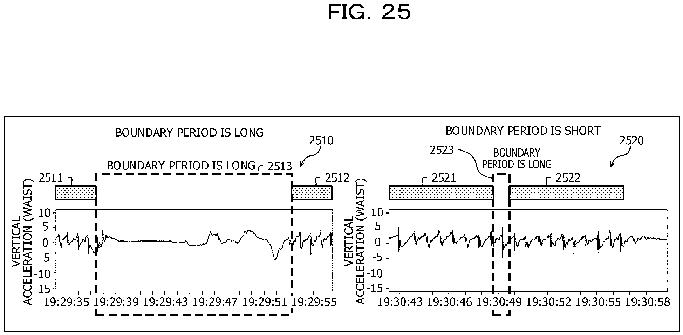

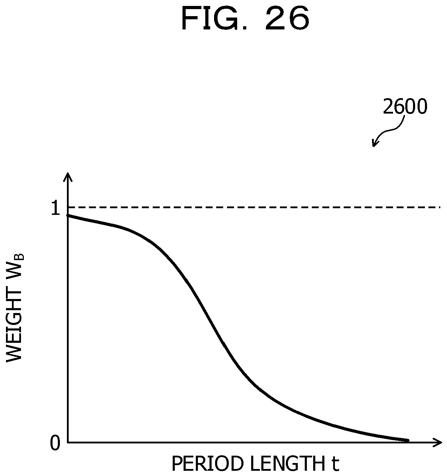

FIGS. 25 and 26 are explanatory diagrams illustrating a fourth example of calculating the likelihood. Here, since an abnormal motion is an exceptional, dangerous motion, a patient tends not to continue the abnormal motion for a long time, and thus a boundary period tends to be short. Accordingly, the length of the boundary period can be one of criteria for evaluating a degree of possibility that an abnormal motion is performed in the boundary period 1030 by the patient. Then, the information processing apparatus 100 calculates, based on the length of the boundary period, the likelihood by the following Formula (5) .

[Mathematical Formula 5]

$$L = w_B \times D_1 \times D_2 \quad ...(5)$$

Here, $w_B$ is the weight set based on the length of the boundary period. For example, as shown in a graph 2510 of FIG. 25, in a case where the length of a boundary period 2513 sandwiched between an earlier walking period 2511 and a later walking period 2512 is relatively long, it is preferable to set a weight $w_B$ small and reduce the likelihood.

On the other hand, as shown in a graph 2520 of FIG. 25, in a case where the length of a boundary period 2523 sandwiched between an earlier walking period 2521 and a later walking period 2522 is relatively short, it is preferable to set the weight $w_B$ large and increase the likelihood. A specific example of setting $w_B$ will be described later with reference to FIG. 26. $D_1$ is dissimilarity of the first feature amounts. $D_2$ is similarity of the second feature amounts. Next, description of FIG. 26 will be made.

A graph 2600 of FIG. 26 shows the weight with respect to the length of a boundary period. The graph 2600 of FIG. 26 is represented by the following Formula (6).

[Mathematical Formula 6]

$$w_B = \frac{1}{1 + exp(\varepsilon_1 t - \varepsilon_2)} \quad ...(6)$$

Here, t is the length of the boundary period. $\varepsilon_1$ and $\varepsilon_2$ are parameters for deciding shapes of functions. $\varepsilon_1$ is negative. The information processing apparatus 100 sets a weight $w_B$ corresponding to the length of the boundary period by using the graph 2600 of FIG. 26, and calculates the likelihood. With this configuration, the information processing apparatus 100 can improve accuracy of calculating likelihood.

(One Example of Determination Result as to Whether or Not Boundary Period is Abnormal Motion Period)

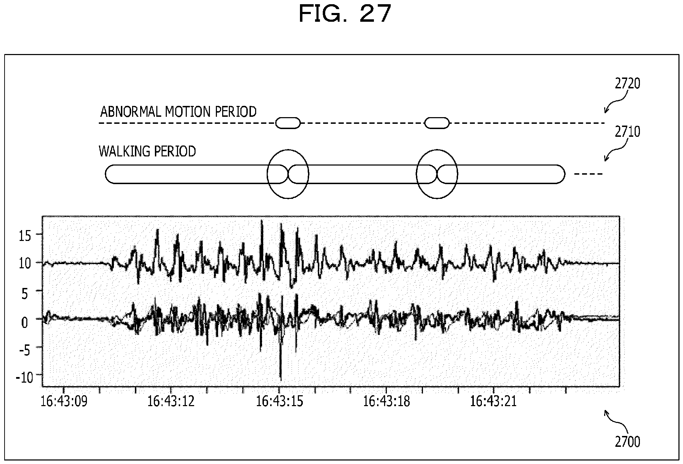

FIGS. 27 to 33 are explanatory diagrams illustrating an example of a determination result as to whether or not a boundary period is an abnormal motion period by the information processing apparatus 100.

A graph 2700 of FIG. 27 is an example of time-series data corresponding to a case where a patient was performing a walking motion. The time-series data includes a vertical acceleration, a horizontal acceleration, and a longitudinal acceleration. A time axis 2710 indicates a period detected as a walking period by a rounded rectangle. A time axis 2720 indicates a period detected as an abnormal motion period by a rounded rectangle.

Based on the time-series data, the information processing apparatus 100 can detect, as a walking period, a period indicated by a rounded rectangle on the time axis 2710. On the other hand, a period circled on the time axis 2710 is not detected as a walking period because the patient temporarily lost his/her balance.

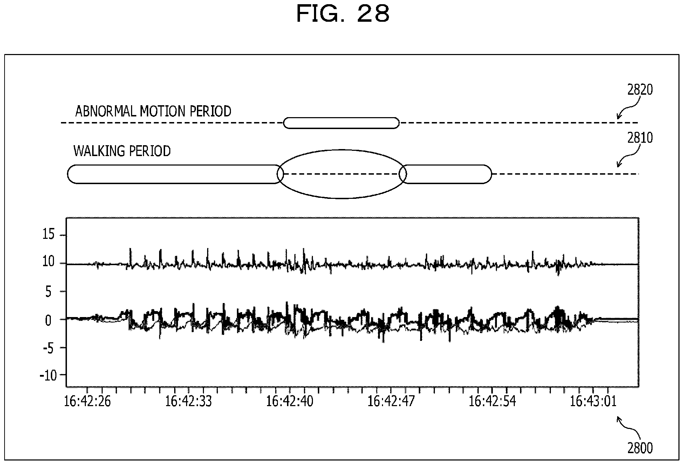

On the other hand, the information processing apparatus 100 can calculate likelihood of an abnormal motion period in which an abnormal motion such as losing balance in a walking motion is performed, as in FIGS. 10 to 26. With this configuration, the information processing apparatus 100 can determine an abnormal motion period in which an abnormal motion related to a walking motion is performed, as shown on the time axis 2720. Therefore, the information processing apparatus 100 can accurately specify a motion period including an abnormal motion period, even if a patient temporarily loses his/her balance. Next, description of FIG. 28 will be made.

A graph 2800 of FIG. 28 is an example of time-series data corresponding to a case where a patient was performing a walking motion. The time-series data includes a vertical acceleration, a horizontal acceleration, and a longitudinal acceleration. A time axis 2810 indicates a period detected as a walking period by a rounded rectangle. A time axis 2820 indicates a period detected as an abnormal motion period by a rounded rectangle.

Based on the time-series data, the information processing apparatus 100 can detect, as a walking period, a period indicated by a rounded rectangle on the time axis 2810. On the other hand, a period circled on the time axis 2810 is not detected as a walking period because the patient lost his/her balance for a certain period.

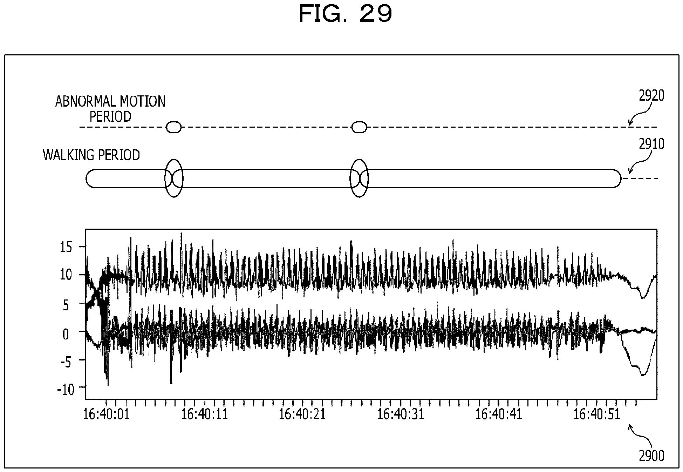

On the other hand, the information processing apparatus 100 can calculate likelihood of an abnormal motion period in which an abnormal motion such as losing balance in a walking motion is performed, as in FIGS. 10 to 26. With this configuration, the information processing apparatus 100 can determine an abnormal motion period in which an abnormal motion related to a walking motion is performed, as shown on the time axis 2820. Therefore, the information processing apparatus 100 can accurately specify a motion period including an abnormal motion period, even if a patient loses his/her balance for a certain period. Next, description of FIG. 29 will be made.

A graph 2900 of FIG. 29 is an example of time-series data corresponding to a case where a patient was performing a walking motion for a long time. The time-series data includes a vertical acceleration, a horizontal acceleration, and a longitudinal acceleration. A time axis 2910 indicates a period detected as a walking period by a rounded rectangle. A time axis 2920 indicates a period detected as an abnormal motion period by a rounded rectangle.

Based on the time-series data, the information processing apparatus 100 can detect, as a walking period, a period indicated by a rounded rectangle on the time axis 2910. On the other hand, a period circled on the time axis 2910 is not detected as a walking period because the patient temporarily lost his/her balance.

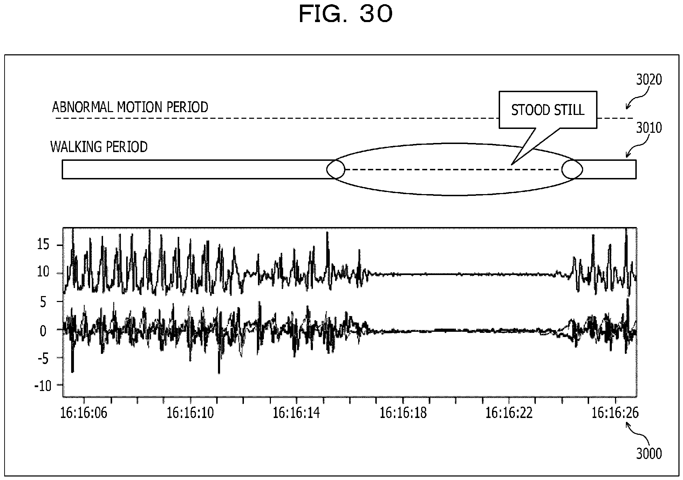

On the other hand, the information processing apparatus 100 can calculate likelihood of an abnormal motion period in which an abnormal motion such as losing balance in a walking motion is performed, as in FIGS. 10 to 26. With this configuration, the information processing apparatus 100 can determine an abnormal motion period in which an abnormal motion related to a walking motion is performed, as shown on the time axis 2920. Therefore, the information processing apparatus 100 can accurately specify a motion period including an abnormal motion period, even if a patient performs a walking motion for a long time and temporarily loses his/her balance. Next, description of FIG. 30 will be made.

A graph 3000 of FIG. 30 is an example of time-series data corresponding to a case where a patient halted while performing a walking motion. The time-series data includes a vertical acceleration, a horizontal acceleration, and a longitudinal acceleration. A time axis 3010 indicates a period detected as a walking period by a rounded rectangle. A time axis 3020 indicates a period detected as an abnormal motion period by a rounded rectangle.

Based on the time-series data, the information processing apparatus 100 can detect, as a walking period, a period indicated by a rounded rectangle on the time axis 3010. On the other hand, a period circled on the time axis 3010 is not detected as a walking period because the patient halted.

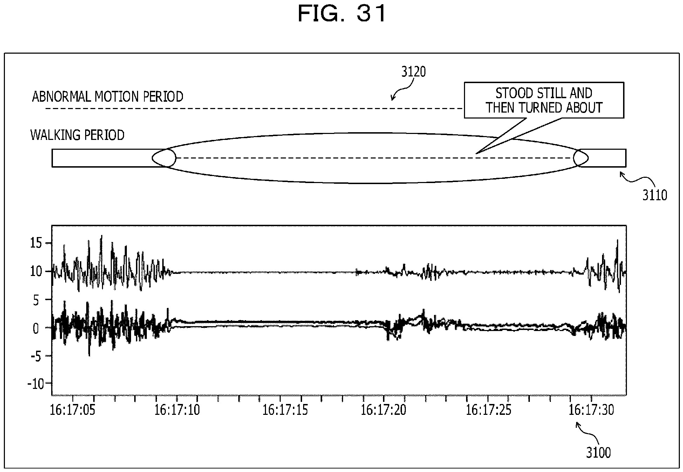

On the other hand, the information processing apparatus 100 can calculate likelihood of an abnormal motion period in which an abnormal motion such as losing balance in a walking motion is performed, as in FIGS. 10 to 26. In the example of FIG. 30, the likelihood is smaller than a threshold. With this configuration, the information processing apparatus 100 can determine that there is no abnormal motion period in which an abnormal motion related to a walking motion is performed, as shown on the time axis 3020. Therefore, the information processing apparatus 100 can prevent a period in which a patient halted while performing a walking motion from being erroneously specified as a motion period. Next, description of FIG. 31 will be made.

A graph 3100 of FIG. 31 is an example of time-series data corresponding to a case where a patient halted and then turned about while performing a walking motion. The time-series data includes a vertical acceleration, a horizontal acceleration, and a longitudinal acceleration. A time axis 3110 indicates a period detected as a walking period by a rounded rectangle. A time axis 3120 indicates a period detected as an abnormal motion period by a rounded rectangle.

Based on the time-series data, the information processing apparatus 100 can detect, as a walking period, a period indicated by a rounded rectangle on the time axis 3110. On the other hand, a period circled on the time axis 3110 is not detected as a walking period because the patient halted and then turned about.

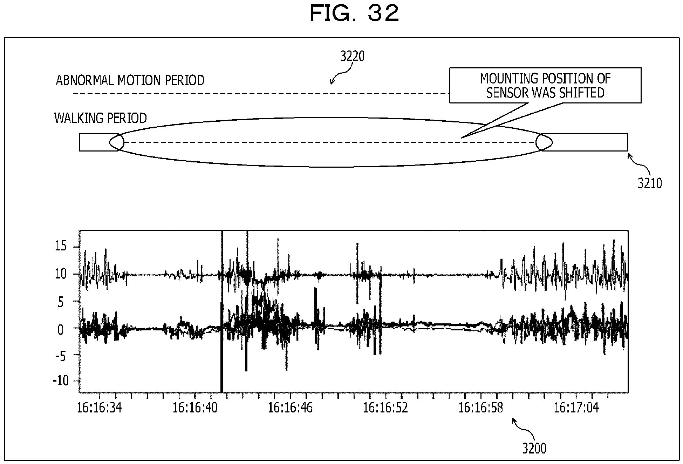

On the other hand, the information processing apparatus 100 can calculate likelihood of an abnormal motion period in which an abnormal motion such as losing balance in a walking motion is performed, as in FIGS. 10 to 26. In the example of FIG. 31, the likelihood is smaller than a threshold. With this configuration, the information processing apparatus 100 can determine that there is no abnormal motion period in which an abnormal motion related to a walking motion is performed, as shown on the time axis 3120. Therefore, the information processing apparatus 100 can prevent a period in which a patient halted and then turned about while performing a walking motion from being erroneously specified as a motion period. Next, description of FIG. 32 will be made.

A graph 3200 of FIG. 32 is an example of time-series data corresponding to a case where a mounting position of the measuring instrument 101 was shifted while a patient was performing a walking motion. The time-series data includes a vertical acceleration, a horizontal acceleration, and a longitudinal acceleration. A time axis 3210 indicates a period detected as a walking period by a rounded rectangle. A time axis 3220 indicates a period detected as an abnormal motion period by a rounded rectangle.

Based on the time-series data, the information processing apparatus 100 can detect, as a walking period, a period indicated by a rounded rectangle on the time axis 3210. On the other hand, a period circled on the time axis 3210 is not detected as a walking period because a mounting position of the measuring instrument 101 was shifted.

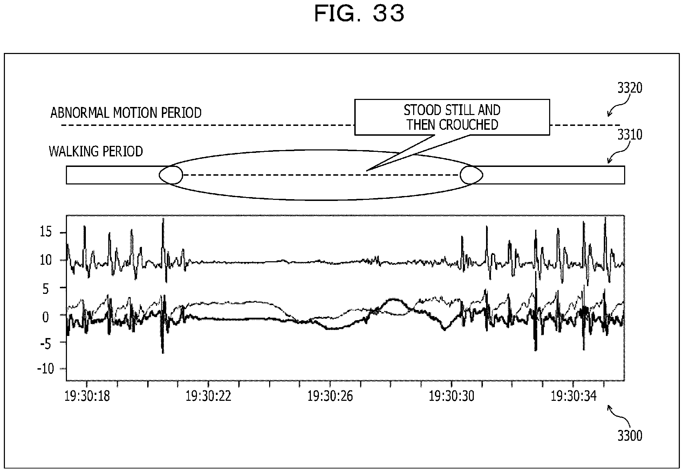

On the other hand, the information processing apparatus 100 can calculate likelihood of an abnormal motion period in which an abnormal motion such as losing balance in a walking motion is performed, as in FIGS. 10 to 26. In the example of FIG. 32, the likelihood is smaller than a threshold. With this configuration, the information processing apparatus 100 can determine that there is no abnormal motion period in which an abnormal motion related to a walking motion is performed, as shown on the time axis 3220. Therefore, the information processing apparatus 100 can prevent a period in which a mounting position of the measuring instrument 101 was shifted from being erroneously specified as a motion period. Next, description of FIG. 33 will be made.

A graph 3300 of FIG. 33 is an example of time-series data corresponding to a case where a patient halted and crouched while performing a walking motion. The time-series data includes a vertical acceleration, a horizontal acceleration, and a longitudinal acceleration. A time axis 3310 indicates a period detected as a walking period by a rounded rectangle. A time axis 3320 indicates a period detected as an abnormal motion period by a rounded rectangle.

Based on the time-series data, the information processing apparatus 100 can detect, as a walking period, a period indicated by a rounded rectangle on the time axis 3310. On the other hand, a period circled on the time axis 3310 is not detected as a walking period because the patient halted and crouched.

On the other hand, the information processing apparatus 100 can calculate likelihood of an abnormal motion period in which an abnormal motion such as losing balance in a walking motion is performed, as in FIGS. 10 to 26. In the example of FIG. 33, the likelihood is smaller than a threshold. With this configuration, the information processing apparatus 100 can determine that there is no abnormal motion period in which an abnormal motion related to a walking motion is performed, as shown on the time axis 3320. Therefore, the information processing apparatus 100 can prevent a period in which a patient halted and crouched from being erroneously specified as a motion period.

In this way, the information processing apparatus 100 can accurately determine whether a boundary period that is not detected as a motion period is an abnormal motion period included in the motion period. Therefore, the information processing apparatus 100 can improve accuracy of specifying a motion period, and support a patient or a medical person such as a doctor.

(One Example of Analysis Processing Procedure)

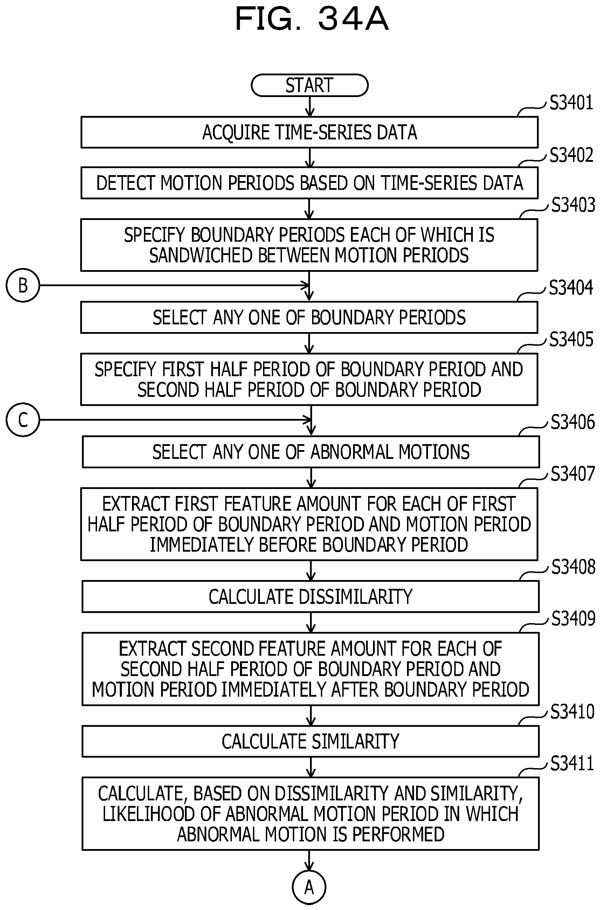

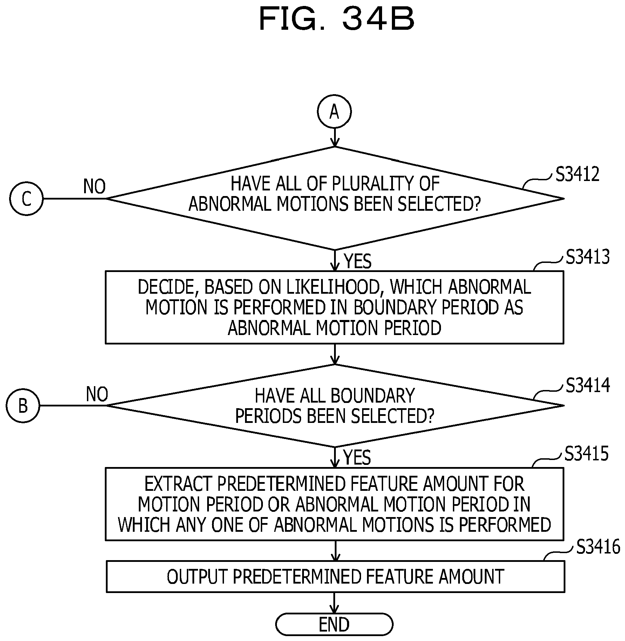

Next, an example of an analysis processing procedure executed by the information processing apparatus 100 will be described with reference to FIG. 34.

FIG. 34 is a flowchart illustrating an example of an analysis processing procedure. In FIG. 34, the information processing apparatus 100 acquires time-series data for a certain period from the measuring instrument 101 (step S3401).

Next, the information processing apparatus 100 detects motion periods in the certain period based on the acquired time-series data (step S3402). Then, based on the detected motion periods, the information processing apparatus 100 specifies boundary periods each of which is sandwiched between the motion periods in the certain period (step S3403).

Next, the information processing apparatus 100 selects any one of the specified boundary periods (step S3404). Then, the information processing apparatus 100 divides the selected boundary period into two, and specifies a first half period of the boundary period and a second half period of the boundary period (step S3405).

Thereafter, the information processing apparatus 100 selects any one of a plurality of abnormal motions (step S3406).

Next, the information processing apparatus 100 extracts a first feature amount for each of the specified first half period of the boundary period and a motion period immediately before the boundary period, in accordance with a determination pattern of the selected abnormal motion (step S3407). Then, the information processing apparatus 100 calculates dissimilarity between the first feature amount extracted for the first half period of the boundary period and the first feature amount extracted for the motion period immediately before the boundary period (step S3408).

Next, the information processing apparatus 100 extracts a second feature amount for each of the specified second half period of the boundary period and a motion period immediately after the boundary period, in accordance with a determination pattern of the selected abnormal motion (step S3409). Then, the information processing apparatus 100 calculates similarity between the second feature amount extracted for the second half period of the boundary period and the second feature amount extracted for the motion period immediately after the boundary period (step S3410).

Thereafter, the information processing apparatus 100 calculates, based on the calculated dissimilarity and similarity, likelihood of an abnormal motion period in which the selected abnormal motion is performed (step S3411).

Next, the information processing apparatus 100 determines whether or not all of the plurality of abnormal motions have been selected (step S3412). Here, in a case where there is an abnormal motion that has not been selected (step S3412: No), the information processing apparatus 100 returns to the processing of step S3406.

On the other hand, in a case where all of the plurality of abnormal motions have been selected (step S3412: Yes), the information processing apparatus 100 proceeds to processing of step S3413. When the processing proceeds to the processing of step S3413, the information processing apparatus 100 decides, based on the likelihood calculated for each abnormal motion, which one of the plurality of abnormal motions is performed in the selected boundary period as the abnormal motion period (step S3413).

Next, the information processing apparatus 100 determines whether or not all of the specified boundary periods have been selected (step S3414). Here, in a case where there is a boundary period that has not been selected (step S3414: No), the information processing apparatus 100 returns to the processing of step S3404.

On the other hand, in a case where all of the boundary periods have been selected (step S3414: Yes), the information processing apparatus 100 extracts a predetermined feature amount for the detected motion period or the abnormal motion period in which any one of the abnormal motions is performed (step S3415).

Next, the information processing apparatus 100 outputs the extracted predetermined feature amount (step S3416). Then, the information processing apparatus 100 ends the analysis processing. With this configuration, the information processing apparatus 100 can accurately specify a motion period, notify a patient or a medical person such as a doctor of an analysis result of the motion period, and easily grasp a condition of the patient.

As described above, according to the information processing apparatus 100, the earlier walking period 1010 in which a motion is performed by a living body and the later walking period 1020 in which a motion is performed by the living body can be detected based on measurement information regarding a movement of the living body. According to the information processing apparatus 100, it is possible to calculate, based on the measurement information, dissimilarity of a first feature amount to the earlier walking period 1010 and similarity of a second feature amount to the later walking period 1020, in the boundary period 1030 between the earlier walking period 1010 and the later walking period 1020. According to the information processing apparatus 100, it is possible to determine whether or not the boundary period 1030 is a third motion period in which a motion is performed by the living body, based on the calculated dissimilarity and similarity. With this configuration, even if it is difficult to detect the boundary period 1030 as a motion period, the information processing apparatus 100 can determine that the boundary period 1030 is the third motion period, and can improve accuracy of specifying various motion periods.

In addition, according to the information processing apparatus 100, a motion period before the boundary period 1030 can be used as the earlier walking period 1010. According to the information processing apparatus 100, a motion period after the boundary period 1030 can be used as the later walking period 1020. According to the information processing apparatus 100, it is possible to calculate, based on the measurement information, dissimilarity of first feature amounts between the first half period 1031 of the boundary period 1030 and the earlier walking period 1010 and similarity of second feature amounts between the second half period 1032 of the boundary period 1030 and the later walking period 1020. With this configuration, the information processing apparatus 100 can improve accuracy of calculating dissimilarity and similarity as one of criteria for evaluating a degree of possibility that an abnormal motion is performed by a living body in the boundary period 1030.

In addition, according to the information processing apparatus 100, it is possible to prepare a plurality of candidate time points for division time points at which the boundary period 1030 is divided into the first half period 1031 and the second half period 1032. According to the information processing apparatus 100, the boundary period 1030 can be divided into two by using a candidate time point at which a ratio of third feature amounts between the first half period 1031 and the second half period 1032 becomes a maximum, among the plurality of candidate time points. With this configuration, the information processing apparatus 100 can improve accuracy of calculating dissimilarity and similarity as one of criteria for evaluating a degree of possibility that an abnormal motion is performed by a living body in the boundary period 1030.

In addition, according to the information processing apparatus 100, likelihood of the third motion period can be calculated so that the greater the dissimilarity, the greater the likelihood, and the greater the similarity, the greater the likelihood. According to the information processing apparatus 100, in a case where the likelihood is equal to or greater than a threshold, the boundary period 1030 can be determined to be the third motion period. With this configuration, the information processing apparatus 100 can compile the dissimilarity and the similarity into the likelihood, and can determine whether or not the boundary period 1030 is the third motion period in consideration of a relationship between a degree of dissimilarity and a degree of similarity. In addition, even if there is an element serving as a criterion for evaluating a degree of possibility that an abnormal motion is performed by a living body in the boundary period 1030, in addition to the dissimilarity and the similarity, the information processing apparatus 100 can compile such an element into the likelihood.

In addition, the information processing apparatus 100 can also be applied to a case of using, as the third motion period, an abnormal motion period in which each of the plurality of abnormal motions is performed. The information processing apparatus 100 can compare likelihood of an abnormal motion period in which each abnormal motion is performed, and determine which abnormal motion is performed in the boundary period 1030 as an abnormal motion period.

In addition, according to the information processing apparatus 100, likelihood can be calculated based on a difference between representative values of the first feature amounts or the second feature amounts in the earlier walking period 1010 and the later walking period 1020. With this configuration, the information processing apparatus 100 can improve accuracy of calculating likelihood.

In addition, according to the information processing apparatus 100, it is possible to calculate likelihood so that the greater an index value indicating a degree of possibility that a motion different from the motions performed in the earlier walking period 1010 and the later walking period 1020 is performed in a part of the boundary period 1030, the smaller the likelihood. With this configuration, the information processing apparatus 100 can improve accuracy of calculating likelihood.

In addition, according to the information processing apparatus 100, likelihood can be calculated so that the longer the boundary period 1030, the smaller the likelihood. With this configuration, the information processing apparatus 100 can improve accuracy of calculating likelihood.

In addition, according to the information processing apparatus 100, dissimilarity can be calculated so that the greater a degree of monotonous change in the first feature amount from the earlier walking period 1010 to the first half period 1031 in a predetermined direction, the greater a value of the dissimilarity. According to the information processing apparatus 100, similarity can be calculated so that the greater a degree of monotonous change in the second feature amount from the second half period 1032 to the later walking period 1020 in a predetermined direction, the greater a value of the similarity. With this configuration, the information processing apparatus 100 can improve accuracy of calculating one of criteria for evaluating a degree of possibility that an abnormal motion is performed by a living body in the boundary period 1030.

In addition, according to the information processing apparatus 100, dissimilarity can be calculated so that the greater the number of times the first feature amount exceeds a predetermined value in the boundary period 1030, the greater a value of the dissimilarity. With this configuration, the information processing apparatus 100 can improve accuracy of calculating one of criteria for evaluating a degree of possibility that an abnormal motion is performed by a living body in the boundary period 1030.

In addition, according to the information processing apparatus 100, the predetermined value can be set based on the representative value of the first feature amount in the earlier walking period 1010. With this configuration, the information processing apparatus 100 can calculate one of criteria for evaluating a degree of possibility that an abnormal motion is performed by a living body in the boundary period 1030 in consideration of a relationship between the first feature amounts of the earlier walking period 1010 and the boundary period 1030.

In addition, according to the information processing apparatus 100, a dispersion value of a synthetic acceleration of a living body can be used as the first feature amount. According to the information processing apparatus 100, a dispersion value of a synthetic acceleration of a living body can be used as the second feature amount. With this configuration, the information processing apparatus 100 can use, as the third motion period, an abnormal motion period in which an abnormal motion such as losing balance in a standing position maintenance motion is performed.

In addition, according to the information processing apparatus 100, a peak amplitude of an acceleration in a vertical direction of a living body can be used as the first feature amount. According to the information processing apparatus 100, a peak interval of an acceleration in a vertical direction of a living body can be used as the second feature amount. With this configuration, the information processing apparatus 100 can use, as the third motion period, an abnormal motion period in which an abnormal motion such as losing balance in a stair climbing motion is performed.

In addition, according to the information processing apparatus 100, a peak amplitude of an acceleration in a longitudinal direction of a living body can be used as the first feature amount. According to the information processing apparatus 100, a peak interval of an acceleration in a longitudinal direction of a living body can be used as the second feature amount. With this configuration, the information processing apparatus 100 can use, as the third motion period, an abnormal motion period in which an abnormal motion such as losing balance in a walking motion is performed.

In addition, according to the information processing apparatus 100, a peak amplitude of an acceleration in a horizontal direction of a living body can be used as the first feature amount. According to the information processing apparatus 100, a dispersion value of an acceleration in a horizontal direction of a living body can be used as the second feature amount. With this configuration, the information processing apparatus 100 can use, as the third motion period, an abnormal motion period in which an abnormal motion such as losing balance in a cycling motion is performed.

Note that the information processing method described in the present embodiment can be implemented by executing a prepared program on a computer such as a personal computer or a workstation. The information processing program described in the present embodiment is recorded on a computer-readable recording medium such as a hard disk, flexible disk, CD-ROM, MO, or DVD, and is read from the recording medium to be executed by the computer. In addition, the information processing program described in the present embodiment may be distributed via a network such as the Internet.

REFERENCE SIGNS LIST

    100 information processing apparatus
    101 measuring instrument
    200 information processing system
    210 network
    300, 400 bus
    301, 401 CPU
    302, 402 memory
    303, 403 network I/F
    304 recording medium I/F
    305 recording medium
    404 sensor unit
    405 timer unit
    500 storage unit
    501 acquisition unit
    502 detection unit
    503 calculation unit
    504 determination unit
    505 output unit
    600 time-series data
    701 to 704 walking period
    801 to 803, 1030, 1730, 2430, 2513, 2523 boundary period
900 determination information table
1000, 1300, 1500, 1610, 1630, 1700, 1900, 2100, 2300, 2400, 2510, 2520, 2600, 2700, 2800, 2900, 3000, 3100, 3200, 3300 graph
1010, 1501, 1611, 1631, 1710, 2410, 2511, 2521 earlier walking period
1020, 1720, 2102, 2420, 2512, 2522 later walking period
1031, 1502, 1612, 1632 first half period
1032, 2101 second half period
1201 to 1205, 1520, 1731 to 1733, 2201, 2203 peak amplitude
1510, 1620, 2110 approximate straight line
1801 to 1806, 2202, 2204 peak interval
2440 seating period
2450 standing period
2710, 2720, 2810, 2820, 2910, 2920, 3010, 3020, 3110, 3120,
3210, 3220, 3310, 3320 time axis

**Claims**

1. An information processing system comprising:

   a measuring instrument; and an information processing apparatus capable of communicating with the measuring instrument,
   the measuring instrument is configured to measure measurement information regarding a movement of a measurement object,
   the information processing apparatus is configured to:

   acquire the measurement information from the measuring instrument,
   detect, based on the acquired measurement information, a first motion period in which a motion is performed by the measurement object and a second motion period in which a motion is performed by the measurement object,
   calculate, based on the acquired measurement information, similarity or dissimilarity of each of one or more feature amounts to the detected first motion period, and similarity or dissimilarity of each of one or more feature amounts to the detected second motion period, in a boundary period between the first motion period and the second motion period, and
   determine, based on a calculation result, whether or not the boundary period is a third motion period in which a motion is performed by the measurement object.

2. The information processing system according to claim 1, wherein

   the first motion period is a motion period before the boundary period,
   the second motion period is a motion period after the boundary period, and
   the information processing apparatus calculates, based on the measurement information, similarity or dissimilarity of each of one or more feature amounts between a first half period of the boundary period and the first motion period, and similarity or dissimilarity of each of one or more feature amounts between a second half period of the boundary period and the second motion period.

3. The information processing system according to claim 2, wherein
   the information processing apparatus divides the boundary period into two using a candidate time point at which a ratio of third feature amounts between the first half period and the second half period becomes a maximum, among a plurality of candidate time points for division time points at which the boundary period is divided into the first half period and the second half period.

4. The information processing system according to any one of claims 1 to 3, wherein
   the information processing apparatus calculates dissimilarity of a first feature amount to the first motion period and similarity of a second feature amount to the second motion period, in the boundary period.

5. The information processing system according to claim 4, wherein
   the information processing apparatus calculates likelihood of the third motion period greater as the dissimilarity is greater, and to make the likelihood greater as the similarity is greater, and determines that the boundary period is the third motion period in a case where the likelihood is equal to or greater than a threshold.

6. The information processing system according to claim 5, wherein
   the information processing apparatus calculates the likelihood based on a difference between representative values of the first feature amount or the second feature amount, in the first motion period and the second motion period.

7. The information processing system according to claim 5 or 6, wherein
   the information processing apparatus calculates the likelihood smaller as an index value is greater, the index value indicating a degree of possibility that a motion different from a motion performed in the first motion period and the second motion period is performed in a part of the boundary period.

8. The information processing system according to any one of claims 5 to 7, wherein
the information processing apparatus calculates the likelihood smaller as the boundary period is longer.

9. The information processing system according to any one of claims 4 to 8, wherein
the information processing apparatus calculates the dissimilarity to make a value of the dissimilarity greater as a degree of monotonous change is greater in the first feature amount from the first motion period to the first half period of the boundary period in a predetermined direction, and calculates the similarity to make a value of the similarity greater as a degree of monotonous change is greater in the second feature amount from the second half period of the boundary period to the second motion period in a predetermined direction.

10. The information processing system according to any one of claims 4 to 8, wherein
the information processing apparatus calculates the dissimilarity to make a value of the dissimilarity greater as the number of times the first feature amount exceeds a predetermined value in the boundary period is greater.

11. The information processing system according to claim 10, wherein
the predetermined value is set based on the representative value of the first feature amount in the first motion period.

12. The information processing system according to any one of claims 1 to 11, wherein
the one or more feature amounts include a peak amplitude, a peak interval, a time interval between zero crossing points, an integral value, or a dispersion value, or any combination thereof for an acceleration, an angular velocity, a position, or terrestrial magnetism, or any combination thereof of the measurement object.

13. The information processing system according to any one of claims 4 to 11, wherein

the first feature amount is a dispersion value of a synthetic acceleration of the measurement object, and
the second feature amount is a dispersion value of a synthetic acceleration of the measurement object.

14. The information processing system according to any one of claims 4 to 11, wherein

the first feature amount is a peak amplitude of an acceleration in a vertical direction of the measurement object, and
the second feature amount is a peak interval of an acceleration in the vertical direction of the measurement object.

15. The information processing system according to any one of claims 4 to 11, wherein

the first feature amount is a peak amplitude of an acceleration in a longitudinal direction of the measurement object, and
the second feature amount is a peak interval of an acceleration in the longitudinal direction of the measurement object.

16. The information processing system according to any one of claims 4 to 11, wherein

the first feature amount is a peak amplitude of an acceleration in a horizontal direction of the measurement object, and
the second feature amount is a dispersion value of an acceleration in the horizontal direction of the measurement object.

17. The information processing system according to claims 4 to 11, wherein
the information processing apparatus outputs, in association with the boundary period determined to be the third motion period, an index value indicating a degree of possibility that a motion different from a motion performed in the first motion period and the second motion period is performed in a part of the boundary period, a length of the boundary period, magnitude of a degree of monotonous change in the first feature amount from the first motion period to the first half period of the boundary period in a predetermined direction, magnitude of a degree of monotonous change in the second feature amount from the second half period of the boundary period to the second motion period in a predetermined direction, or the number of times the first feature amount exceeds a predetermined value in the boundary period, or any combination thereof.

18. The information processing system according to claims 1 to 17, wherein
the information processing apparatus outputs, in association with the boundary period determined to be the third motion period, a predetermined feature amount in the boundary period determined to be the third motion period.

19. The information processing system according to claims 1 to 18, wherein
the information processing apparatus outputs, in association with the first motion period and the second motion period, predetermined feature amounts in the first motion period and the second motion period.

20. The information processing system according to claim 18 or 19, wherein
the predetermined feature amount includes a peak amplitude, a peak interval, a time interval between zero crossing points, an integral value, or a dispersion value for an acceleration, an angular velocity, a position, or terrestrial magnetism of the measurement object.

21. The information processing system according to claims 1 to 20, wherein
the information processing apparatus outputs, in association with the boundary period determined to be the third motion period, a calculation result or a determination result, or any combination thereof.

22. An information processing apparatus comprising a control unit configured to:

acquire measurement information regarding a movement of a measurement object;
detect, based on the acquired measurement information, a first motion period in which a motion is performed by the measurement object and a second motion period in which a motion is performed by the measurement object;
calculate, based on the acquired measurement information, similarity or dissimilarity of each of one or more feature amounts to the detected first motion period and similarity or dissimilarity of each of one or more feature amounts to the detected second motion period, in a boundary period between the first motion period and the second motion period; and
determine, based on a calculation result, whether or not the boundary period is a third motion period in which a motion is performed by the measurement object.

23. An information processing method for causing a computer to execute processing comprising:

acquiring measurement information regarding a movement of a measurement object;
detecting, based on the acquired measurement information, a first motion period in which a motion is performed by the measurement object and a second motion period in which a motion is performed by the measurement object;
calculating, based on the acquired measurement information, similarity or dissimilarity of each of one or more feature amounts to the detected first motion period and similarity or dissimilarity of each of one or more feature amounts to the detected second motion period, in a boundary period between the first motion period and the second motion period; and
determining, based on a calculation result, whether or not the boundary period is a third motion period in which a motion is performed by the measurement object.

# FIG. 1

# FIG. 2

# FIG. 3

# FIG. 4

FIG. 5

# FIG. 6

ACCELERATION OF WAIST IN
LONGITUDINAL DIRECTION

ACCELERATION OF WAIST
IN VERTICAL DIRECTION

600

VERTICAL ACCELERATION
(WAIST)

15
10
5
0
-5
-10

19:52:14    19:52:19    19:52:24    19:52:29    19:52:34    19:52:39

TIME

ACCELERATION OF WAIST IN
HORIZONTAL DIRECTION

FIG. 7

FIG. 8

# FIG. 9

900

| BOUNDARY PERIOD NUMBER | LIKELIHOOD OF ABNORMAL MOTION PERIOD | DETERMINATION RESULT |
|---|---|---|
| No1 | 0.89 | ○ |
| No2 | 0.01 | × |
| No3 | 0.65 | ○ |

# FIG. 10

# FIG. 11

## FIG. 12

| FIRST FEATURE AMOUNT IN EARLIER WALKING PERIOD | PEAK NUMBER | 1 | 2 | 3 |
|---|---|---|---|---|
| | PEAK AMPLITUDE | 3.2 | 2.8 | 2.9 |

| FIRST FEATURE AMOUNT IN FIRST HALF PERIOD | PEAK NUMBER | 1 | 2 |
|---|---|---|---|
| | PEAK AMPLITUDE | 7.4 | 1.6 |

# FIG. 13

FIG. 14

| | |
|---|---|
| MAXIMUM VALUE OF FIRST FEATURE AMOUNT IN FIRST HALF PERIOD | 7.4 |
| MAXIMUM VALUE OF FIRST FEATURE AMOUNT IN EARLIER WALKING PERIOD | 3.2 |
| RATIO OF FIRST FEATURE AMOUNTS | 2.3 |
| DISSIMILARITY | 0.87 |

# FIG. 15

1500

1501
EARLIER WALKING PERIOD

1502
FIRST HALF PERIOD

APPROXIMATE STRAIGHT LINE
1510

FIRST FEATURE AMOUNT

TIME

1520

FIG. 16

# FIG. 17

VERTICAL ACCELERATION (WAIST)

BOUNDARY PERIOD ⌐1730

1710    1720    1700

10
5
0
-5
-10
-15

1731    1733
        1732

12:13:07    12:13:10    12:13:13    12:13:16    12:13:19

| FIRST FEATURE AMOUNT IN BOUNDARY PERIOD | PEAK NUMBER | 1 | 2 | 3 | 4 | ⋯ |
|---|---|---|---|---|---|---|
| | PEAK AMPLITUDE | 5.4 | 6.8 | 7.0 | 1.8 | ⋯ |
| | PREDETERMINED VALUE Fth | 4.0 | 4.0 | 4.0 | 4.0 | ⋯ |
| | DETERMINE MAGNITUDE OF PREDETERMINED VALUE Fth | ○ | ○ | ○ | × | |

CONTINUED FOR MORE THAN OR EQUAL TO
THE PREDETERMINED NUMBER OF TIMES

## FIG. 18

| SECOND FEATURE AMOUNT IN SECOND HALF PERIOD | PEAK NUMBER | 1 | 2 | 3 |
|---|---|---|---|---|
| | PEAK INTERVAL | 0.85 | 0.68 | 0.65 |

| SECOND FEATURE AMOUNT IN LATER WALKING PERIOD | PEAK NUMBER | 1 | 2 | 3 |
|---|---|---|---|---|
| | PEAK INTERVAL | 0.65 | 0.63 | 0.61 |

# FIG. 19

FIG. 20

| | |
|---|---|
| MAXIMUM VALUE OF SECOND FEATURE AMOUNT IN SECOND HALF PERIOD | 0.63 |
| MAXIMUM VALUE OF SECOND FEATURE AMOUNT IN LATER WALKING PERIOD | 0.73 |
| RATIO OF SECOND FEATURE AMOUNTS | 0.86 |
| SIMILARITY | 0.78 |

# FIG. 21

# FIG. 22

# FIG. 23

# FIG. 24

FIG. 25

# FIG. 26

## FIG. 27

ABNORMAL MOTION PERIOD    2720

WALKING PERIOD    2710

2700

# FIG. 28

# FIG. 29

ABNORMAL MOTION PERIOD                                                    2920

WALKING PERIOD                                                           2910

2900

# FIG. 30

# FIG. 31

ABNORMAL MOTION PERIOD

3120

WALKING PERIOD

STOOD STILL AND
THEN TURNED ABOUT

3110

## FIG. 32

# FIG. 33

# FIG. 34A

START

ACQUIRE TIME-SERIES DATA — S3401

DETECT MOTION PERIODS BASED ON TIME-SERIES DATA — S3402

SPECIFY BOUNDARY PERIODS EACH OF WHICH IS SANDWICHED BETWEEN MOTION PERIODS — S3403

(B) → SELECT ANY ONE OF BOUNDARY PERIODS — S3404

SPECIFY FIRST HALF PERIOD OF BOUNDARY PERIOD AND SECOND HALF PERIOD OF BOUNDARY PERIOD — S3405

(C) → SELECT ANY ONE OF ABNORMAL MOTIONS — S3406

EXTRACT FIRST FEATURE AMOUNT FOR EACH OF FIRST HALF PERIOD OF BOUNDARY PERIOD AND MOTION PERIOD IMMEDIATELY BEFORE BOUNDARY PERIOD — S3407

CALCULATE DISSIMILARITY — S3408

EXTRACT SECOND FEATURE AMOUNT FOR EACH OF SECOND HALF PERIOD OF BOUNDARY PERIOD AND MOTION PERIOD IMMEDIATELY AFTER BOUNDARY PERIOD — S3409

CALCULATE SIMILARITY — S3410

CALCULATE, BASED ON DISSIMILARITY AND SIMILARITY, LIKELIHOOD OF ABNORMAL MOTION PERIOD IN WHICH ABNORMAL MOTION IS PERFORMED — S3411

(A)

# FIG. 34B

(A)

HAVE ALL OF PLURALITY OF
ABNORMAL MOTIONS BEEN SELECTED? — S3412

NO — (C)

↓YES

DECIDE, BASED ON LIKELIHOOD, WHICH ABNORMAL
MOTION IS PERFORMED IN BOUNDARY PERIOD AS
ABNORMAL MOTION PERIOD — S3413

HAVE ALL BOUNDARY
PERIODS BEEN SELECTED? — S3414

NO — (B)

↓YES

EXTRACT PREDETERMINED FEATURE AMOUNT FOR
MOTION PERIOD OR ABNORMAL MOTION PERIOD IN
WHICH ANY ONE OF ABNORMAL MOTIONS IS PERFORMED — S3415

OUTPUT PREDETERMINED FEATURE AMOUNT — S3416

END

## INTERNATIONAL SEARCH REPORT

| International application No. |
|---|
| PCT/JP2017/013485 |

A. CLASSIFICATION OF SUBJECT MATTER
*A61B5/11(2006.01)i*

According to International Patent Classification (IPC) or to both national classification and IPC

B. FIELDS SEARCHED

Minimum documentation searched (classification system followed by classification symbols)
A61B5/06-5/22

Documentation searched other than minimum documentation to the extent that such documents are included in the fields searched
Jitsuyo Shinan Koho         1922–1996   Jitsuyo Shinan Toroku Koho   1996–2017
Kokai Jitsuyo Shinan Koho   1971–2017   Toroku Jitsuyo Shinan Koho   1994–2017

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 2016-220923 A (GE Medical Systems Global Technology Co., L.L.C.), 28 December 2016 (28.12.2016), paragraphs [0026] to [0158]; fig. 1 to 28 (Family: none) | 1-23 |
| A | JP 2015-139669 A (GE Medical Systems Global Technology Co., L.L.C.), 03 August 2015 (03.08.2015), paragraphs [0031] to [0167]; fig. 1 to 35 (Family: none) | 1-23 |
| A | JP 2013-59489 A (Toshiba Corp.), 04 April 2013 (04.04.2013), paragraphs [0012] to [0054]; fig. 1 to 9 (Family: none) | 1-23 |

☐ Further documents are listed in the continuation of Box C.        ☐ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 16 June 2017 (16.06.17) | 04 July 2017 (04.07.17) |

| Name and mailing address of the ISA/ | Authorized officer |
|---|---|
| Japan Patent Office 3-4-3,Kasumigaseki,Chiyoda-ku, Tokyo 100-8915,Japan | Telephone No. |

Form PCT/ISA/210 (second sheet) (January 2015)

**EP 3 603 506 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2015139667 A
- JP 2013059489 A
- JP 2011188901 A
- JP 2008171347 A